# EUROPEAN PATENT SPECIFICATION

(11) **EP 3 325 015 B1**
(45) Date of publication and mention of the grant of the patent: **12.05.2021**
(21) Application number: 16745318.2
(22) Date of filing: 19.07.2016
(51) Int. Cl.: A61K 47/18, A61K 47/24, A61K 47/28, A61K 9/127

(54) **LIPOSOMAL ADJUVANT COMPOSITIONS**
LIPOSOMALE HILFSSTOFFZUSAMMENSETZUNGEN
COMPOSITIONS D'ADJUVANT LIPOSOMAL

(30) Priority: 20.07.2015 US 201562194355 P
(43) Date of publication of application: 30.05.2018
(73) Proprietor: Zoetis Services LLC, Parsippany, NJ 07054 (US)
(72) Inventor: DOMINOWSKI, Paul, Joseph, Kalamazoo, MI 49007 (US); MWANGI, Duncan, Kalamazoo, MI 49007 (US); RAI, Sharath, K., Kalamazoo, MI 49007 (US); FOSS, Dennis, L., Kalamazoo, MI 49007 (US); GODBEE, Traci, K., Kalamazoo, MI 49007 (US); SLY, Laurel, Mary, Kalamazoo, MI 49007 (US); MAHAN, Suman, Kalamazoo, MI 49007 (US); VORA, Shaunak, Kalamazoo, MI 49007 (US)
(74) Representative: Mannion, Sally Kim
(86) International application number: PCT/US2016/042882
(87) International publication number: WO 2017/015252

(56) References cited:
- AU-B2- 750 379
- AU-B2- 750 379
- US-A1- 2006 008 519

### SEQUENCE LISTING

<110> Zoetis Services LLC
   Dominowski, Paul J
   Mwangi, Duncan
   Rai, Sharath K
   Foss, Dennis L
   Godbee, Traci K
   Sly, Laurel M
   Mahan, Suman
   Vora, Shaunak
<120> Liposomal Adjuvant Compositions
<130> ZP000111
<150> US 62/194,355
   <151> 2015-07-20
<160> 136
<170> Patent In version 3.5
<210> 1
   <211> 23
   <212> DNA
   <213> Artificial Sequence
<220>
   <223> CpG oligonucleotide
<400> 1
   tcgtcgacga tcggcgcgcg ccg 23
<210> 2
   <211> 23
   <212> DNA
   <213> Artificial sequence
<220>
   <223> CpG oligonucleotide
<400> 2
   tcgacgtcga tcggcgcgcg ccg 23
<210> 3
   <211> 24
   <212> DNA
   <213> Artificial Sequence
<220>
   <223> CpG oligonucleotide
<400> 3
   tcgacgtcga tcggcgcgcg ccgt 24
<210> 4
   <211> 23
   <212> DNA
   <213> Artificial Sequence
<220>
   <223> CpG oligonucleotide
<220>
   <221> misc_feature
   <222> (1)..(1)
   <223> 5'-Iodo-2'-deoxyuridine
<400> 4
   ncgacgtcga tcggcgcgcg ccg 23
<210> 5
   <211> 24
   <212> DNA
   <213> Artificial Sequence
<220>
   <223> CpG oligonucleotide
<220>
   <221> misc_feature
   <222> (1)..(1)
   <223> 5'-Iodo-2'-deoxyuridine
<400> 5
   ncgacgtcga tcggcgcgcg ccgt 24
<210> 6
   <211> 24
   <212> DNA
   <213> Artificial Sequence
<220>
   <223> CpG Oligonucleotide
<220>
   <221> miac_feature
   <222> (1)..(1)
   <223> 5'-Iodo-2'-deoxyuridine
<400> 6
   ncgacgtcga tcggcgcgcg ccgt 24
<210> 7
   <211> 23
   <212> DNA
   <213> Artificial Sequence
<220>
   <223> CpG oligonucleotide
<220>
   <221> misc_feature
   <222> (1)..(1)
   <223> 5'-Ethyl-2'-deoxyuridine
<400> 7
   ncgacgtcga tcggcgcgcg ccg 23
<210> 8
   <211> 24
   <212> DNA
   <213> Artificial Sequence
<220>
   <223> CpG Oligonucleotide
<220>
   <221> misc_feature
   <222> (1)..(1)
   <223> 5'-Iodo-2'-deoxyuridine
<400> 8
   ncgtcgacga tcggcggccg ccgt 24
<210> 9
   <211> 24
   <212> DNA
   <213> Artificial Sequence
<220>
   <223> CpG Oligonucleotide
<220>
   <221> miac_feature
   <222> (1)..(1)
   <223> 5'-Iodo-2'-deoxyuridine
<400> 9
   ncgtcgacga tcggcggccg ccgt 24
<210> 10
   <211> 23
   <212> DNA
   <213> Artificial Sequence
<220>
   <223> CpG Oligonucleotide
<400> 10
   tcgtcgacga tcggcgcgcg ccg 23
<210> 11
   <211> 20
   <212> RNA
   <213> Artificial Sequence
<220>
   <223> imunostimulatory oligoribonucleotide
<400> 11
   uuguuguugu uguuguuguu 20
<210> 12
   <211> 20
   <212> RNA
   <213> Artificial Sequence
<220>
   <223> Immunostimulatory oligoribonucleotide
<400> 12
   uuauuauuau uauuauuauu 20
<210> 13
   <211> 19
   <212> RNA
   <213> Artificial Sequence
<220>
   <223> Immunostimulatory oligoribonucleotide
<400> 13
   aaacgcucag ccaaagcag 19
<210> 14
   <211> 21
   <212> DNA
   <213> Artificial Sequence
<220>
   <223> DNA/RNA hybrid
<220>
   <221> misc_feature
   <222> (11)..(17)
   <223> ribonucleotides
<400> 14
   tcgtcgtttt guuguguttt t 21
<210> 15
   <211> 16
   <212> PRT
   <213> Artificial Sequence
<220>
   <223> Fragment of Swine Influenza Virus NP protein
<400> 15
   Met Ala Thr Gln Gly Thr Lys Arg Ser Tyr Glu Gln Met Glu Thr Gly
   1               5                   10                  15
<210> 16
   <211> 16
   <212> PRT
   <213> Artificial sequence
<220>
   <223> Fragment of NP protein of Swine Influenza Virus
<400> 16
   Gly Thr Lys Arg Ser Tyr Glu Gln Met Glu Thr Gly Gly Glu Arg Gln
   1               5                   10                  15
<210> 17
   <211> 16
   <212> PRT
   <213> Artificial Sequence
<220>
   <223> Fragment of Swine Influenza Virus NP protein
<400> 17
   Ser Tyr Glu Gln Met Glu Thr Gly Gly Glu Arg Gln Asp Ala Thr Glu
   1               5                   10                  15
<210> 18
   <211> 16
   <212> PRT
   <213> Artificial Sequence
<220>
   <223> Fragment of NP protein of Swine Influenza Virus
<400> 18
   Met Glu Thr Gly Gly Glu Arg Gln Asp Ala Thr Glu Ile Lys Ala Ser
   1               5                   10                  15
<210> 19
   <211> 16
   <212> PRT
   <213> Artificial Sequence
<220>
   <223> Fragment of NP protein of Swine Influenza Virus
<400> 19
   Gly Glu Arg Gln Asp Ala Thr Glu Ile Lys Ala Ser Val Gly Arg Met
   1               5                   10                  15
<210> 20
   <211> 16
   <212> PRT
   <213> Artificial Sequence
<220>
   <223> Fragment of NP protein of Swine Influenza Virus
<400> 20
   Asp Ala Thr Glu Ile Lys Ala Ser Val Gly Arg Met Val Gly Gly Ile
   1               5                   10                  15
<210> 21
   <211> 16
   <212> PRT
   <213> Artificial Sequence
<220>
   <223> Fragment of NP protein of Swine Influenza Virus
<400> 21
   Ile Lys Ala Ser Val Gly Arg Met Val Gly Gly Ile Gly Arg Phe Tyr
   1               5                   10                  15
<210> 22
   <211> 16
   <212> PRT
   <213> Artificial Sequence
<220>
   <223> Fragment of NP protein of Swine Influenza Virus
<400> 22
   Val Gly Arg Met Val Gly Gly Ile Gly Arg Phe Tyr Ile Gln Met Cys
   1               5                   10                  15
<210> 23
   <211> 16
   <212> PRT
   <213> Artificial Sequence
<220>
   <223> Fragment of NP protein of Swine Influenza Virus
<400> 23
   Val Gly Gly Ile Gly Arg Phe Tyr Ile Gln Met Cys Thr Glu Leu Lys
   1               5                   10                  15
<210> 24
   <211> 16
   <212> PRT
   <213> Artificial Sequence
<220>
   <223> Fragment of NP protein of Swine Influenza Virus
<400> 24
   Gly Arg Phe Tyr Ile Gln Met Cys Thr Glu Leu Lys Leu Ser Asp Tyr
   1               5                   10                  15
<210> 25
   <211> 16
   <212> PRT
   <213> Artificial Sequence
<220>
   <223> Fragment of NP protein of Swine Influenza Virus
<400> 25
   Ile Gln Met Cys Thr Glu Leu Lys Leu Ser Asp Tyr Glu Gly Arg Leu
   1               5                   10                  15
<210> 26
   <211> 16
   <212> PRT
   <213> Artificial Sequence
<220>
   <223> Fragment of NP protein of Swine Influenza Virus
<400> 26
   Thr Glu Leu Lys Leu Ser Asp Tyr Glu Gly Arg Leu Ile Gln Asn Ser
   1               5                   10                  15
<210> 27
   <211> 16
   <212> PRT
   <213> Artificial Sequence
<220>
   <223> Fragment of NP protein of Swine Influenza Virus
<400> 27
   Leu Ser Asp Tyr Glu Gly Arg Leu Ile Gln Asn Ser Ile Thr Ile Glu
   1               5                   10                  15
<210> 28
   <211> 16
   <212> PRT
   <213> Artificial Sequence
<220>
   <223> Fragment of NP protein of Swine Influenza Virus
<400> 28
   Glu Gly Arg Leu Ile Gln Asn Ser Ile Thr Ile Glu Arg Met Val Leu
   1               5                   10                  15
<210> 29
   <211> 16
   <212> PRT
   <213> Artificial Sequence
<220>
   <223> Fragment of NP protein of Swine Influenza Virus
<400> 29
   Ile Gln Asn Ser Ile Thr Ile Glu Arg Met Val Leu Ser Ala Phe Asp
   1               5                   10                  15
<210> 30
   <211> 16
   <212> PRT
   <213> Artificial Sequence
<220>
   <223> Fragment of NP protein of Swine Influenza Virus
<400> 30
   Ile Thr Ile Glu Arg Met Val Leu Ser Ala Phe Asp Glu Arg Arg Asn
   1               5                   10                  15
<210> 31
   <211> 16
   <212> PRT
   <213> Artificial Sequence
<220>
   <223> Fragment of NP protein of Swine Influenza Virus
<400> 31
   Arg Met Val Leu Ser Ala Phe Asp Glu Arg Arg Asn Lys Tyr Leu Glu
   1               5                   10                  15
<210> 32
   <211> 16
   <212> PRT
   <213> Artificial Sequence
<220>
   <223> Fragment of NP protein of Swine Influenza Virus
<400> 32
   Ser Ala Phe Asp Glu Arg Arg Asn Lys Tyr Leu Glu Glu His Pro Ser
   1               5                   10                  15
<210> 33
   <211> 16
   <212> PRT
   <213> Artificial Sequence
<220>
   <223> Fragment of NP protein of Swine Influenza Virus
<400> 33
   Glu Arg Arg Asn Lys Tyr Leu Glu Glu His Pro Ser Ala Gly Lys Asp
   1               5                   10                  15
<210> 34
   <211> 16
   <212> PRT
   <213> Artificial Sequence
<220>
   <223> Fragment of NP protein of Swine Influenza Virus
<400> 34
   Lys Tyr Leu Glu Glu His Pro Ser Ala Gly Lys Asp Pro Lys Lys Thr
   1               5                   10                  15
<210> 35
   <211> 16
   <212> PRT
   <213> Artificial Sequence
<220>
   <223> Fragment of NP protein of Swine Influenza Virus
<400> 35
   Glu His Pro Ser Ala Gly Lys Asp Pro Lys Lys Thr Gly Gly Pro Ile
   1 5 10 15
<210> 36
   <211> 16
   <212> PRT
   <213> Artificial Sequence
<220>
   <223> Fragment of NP protein of Swine Influenza Virus
<400> 36
   Ala Gly Lys Asp Pro Lys Lys Thr Gly Gly Pro Ile Tyr Arg Arg Val
   1 5 10 15
<210> 37
   <211> 16
   <212> PRT
   <213> Artificial Sequence
<220>
   <223> Fragment of NP protein of Swine Influenza Virus
<400> 37
   Pro Lys Lys Thr Gly Gly Pro Ile Tyr Arg Arg Val Asp Gly Lys Trp
   1 5 10 15
<210> 38
   <211> 16
   <212> PRT
   <213> Artificial Sequence
<220>
   <223> Fragment of NP protein of Swine Influenza Virus
<400> 38
   Gly Gly Pro Ile Tyr Arg Arg Val Asp Gly Lys Trp Met Arg Glu Leu
   1 5 10 15
<210> 39
   <211> 16
   <212> PRT
   <213> Artificial Sequence
<220>
   <223> Fragment of NP protein of Swine Influenza Virus
<400> 39
   Tyr Arg Arg Val Asp Gly Lys Trp Met Arg Glu Leu Ile Leu Tyr Asp
   1 5 10 15
<210> 40
   <211> 16
   <212> PRT
   <213> Artificial Sequence
<220>
   <223> Fragment of NP protein of Swine Influenza Virus
<400> 40
   Asp Gly Lys Trp Met Arg Glu Leu Ile Leu Tyr Asp Lys Glu Glu Ile
   1               5                   10                  15
<210> 41
   <211> 16
   <212> PRT
   <213> Artificial Sequence
<220>
   <223> Fragment of NP protein of Swine Influenza Virus
<400> 41
   Met Arg Glu Leu Ile Leu Tyr Asp Lys Glu Glu Ile Arg Arg Val Trp
   1               5                   10                  15
<210> 42
   <211> 16
   <212> PRT
   <213> Artificial Sequence
<220>
   <223> Fragment of NP protein of Swine Influenza Virus
<400> 42
   Ile Leu Tyr Asp Lys Glu Glu Ile Arg Arg Val Trp Arg Gln Ala Asn
   1               5                   10                  15
<210> 43
   <211> 16
   <212> PRT
   <213> Artificial Sequence
<220>
   <223> Fragment of NP protein of Swine Influenza Virus
<400> 43
   Lys Glu Glu Ile Arg Arg Val Trp Arg Gln Ala Asn Asn Gly Glu Asp
   1               5                   10                  15
<210> 44
   <211> 16
   <212> PRT
   <213> Artificial Sequence
<220>
   <223> Fragment of NP protein of Swine Influenza Virus
<400> 44
   Arg Arg Val Trp Arg Gln Ala Asn Asn Gly Glu Asp Ala Thr Ala Gly
   1 5 10 15
<210> 45
   <211> 16
   <212> PRT
   <213> Artificial Sequence
<220>
   <223> Fragment of NP protein of Swine Influenza Virus
<400> 45
   Arg Gln Ala Asn Asn Gly Glu Asp Ala Thr Ala Gly Leu Thr His Ile
   1 5 10 15
<210> 46
   <211> 16
   <212> PRT
   <213> Artificial Sequence
<220>
   <223> Fragment of NP protein of Swine Influenza Virus
<400> 46
   Asn Gly Glu Asp Ala Thr Ala Gly Leu Thr His Ile Met Ile Trp His
   1 5 10 15
<210> 47
   <211> 16
   <212> PRT
   <213> Artificial Sequence
<220>
   <223> Fragment of NP protein of Swine Influenza Virus
<400> 47
   Ala Thr Ala Gly Leu Thr His Ile Met Ile Trp His Ser Asn Leu Asn
   1 5 10 15
<210> 48
   <211> 16
   <212> PRT
   <213> Artificial Sequence
<220>
   <223> Fragment of NP protein of Swine Influenza Virus
<400> 48
   Leu Thr His Ile Met Ile Trp His Ser Asn Leu Asn Asp Ala Thr Tyr
   1 5 10 15
<210> 49
   <211> 16
   <212> PRT
   <213> Artificial Sequence
<220>
   <223> Fragment of NP protein of Swine Influenza Virus
<400> 49
   Met Ile Trp His Ser Asn Leu Asn Asp Ala Thr Tyr Gln Arg Thr Arg
   1               5                   10                  15
<210> 50
   <211> 16
   <212> PRT
   <213> Artificial Sequence
<220>
   <223> Fragment of NP protein of Swine Influenza Virus
<400> 50
   Ser Asn Leu Asn Asp Ala Thr Tyr Gln Arg Thr Arg Ala Leu Val Arg
   1               5                   10                  15
<210> 51
   <211> 16
   <212> PRT
   <213> Artificial Sequence
<220>
   <223> Fragment of NP protein of Swine Influenza Virus
<400> 51
   Asp Ala Thr Tyr Gln Arg Thr Arg Ala Leu Val Arg Thr Gly Met Asp
   1               5                   10                  15
<210> 52
   <211> 16
   <212> PRT
   <213> Artificial Sequence
<220>
   <223> Fragment of NP protein of Swine Influenza Virus
<400> 52
   Gln Arg Thr Arg Ala Leu Val Arg Thr Gly Met Asp Pro Arg Met Cys
   1               5                   10                  15
<210> 53
   <211> 16
   <212> PRT
   <213> Artificial Sequence
<220>
   <223> Fragment of NP protein of Swine Influenza Virus
<400> 53
   Ala Leu Val Arg Thr Gly Met Asp Pro Arg Met Cys Ser Leu Met Gln
   1 5 10 15
<210> 54
   <211> 16
   <212> PRT
   <213> Artificial Sequence
<220>
   <223> Fragment of NP protein of Swine Influenza Virus
<400> 54
   Thr Gly Met Asp Pro Arg Met Cys Ser Leu Met Gln Gly Ser Thr Leu
   1 5 10 15
<210> 55
   <211> 16
   <212> PRT
   <213> Artificial Sequence
<220>
   <223> Fragment of NP protein of Swine Influenza Virus
<400> 55
   Pro Arg Met Cys Ser Leu Met Gln Gly Ser Thr Leu Pro Arg Arg Ser
   1 5 10 15
<210> 56
   <211> 16
   <212> PRT
   <213> Artificial Sequence
<220>
   <223> Fragment of NP protein of Swine Influenza Virus
<400> 56
   Ser Leu Met Gln Gly Ser Thr Leu Pro Arg Arg Ser Gly Ala Ala Gly
   1 5 10 15
<210> 57
   <211> 16
   <212> PRT
   <213> Artificial Sequence
<220>
   <223> Fragment of NP protein of Swine Influenza Virus
<400> 57
   Gly Ser Thr Leu Pro Arg Arg Ser Gly Ala Ala Gly Ala Ala Val Lys
   1 5 10 15
<210> 58
   <211> 16
   <212> PRT
   <213> Artificial Sequence
<220>
   <223> Fragment of NP protein of Swine Influenza Virus
<400> 58
   Pro Arg Arg Ser Gly Ala Ala Gly Ala Ala Val Lys Gly Val Gly Thr
   1               5                   10                  15
<210> 59
   <211> 16
   <212> PRT
   <213> Artificial Sequence
<220>
   <223> Fragment of NP protein of Swine Influenza Virus
<400> 59
   Gly Ala Ala Gly Ala Ala Val Lys Gly Val Gly Thr Ile Ala Met Glu
   1               5                   10                  15
<210> 60
   <211> 16
   <212> PRT
   <213> Artificial Sequence
<220>
   <223> Fragment of NP protein of Swine Influenza Virus
<400> 60
   Ala Ala Val Lys Gly Val Gly Thr Ile Ala Met Glu Leu Ile Arg Met
   1               5                   10                  15
<210> 61
   <211> 16
   <212> PRT
   <213> Artificial Sequence
<220>
   <223> Fragment of NP protein of Swine Influenza Virus
<400> 61
   Gly Val Gly Thr Ile Ala Met Glu Leu Ile Arg Met Ile Lys Arg Gly
   1               5                   10                  15
<210> 62
   <211> 16
   <212> PRT
   <213> Artificial Sequence
<220>
   <223> Fragment of NP protein of Swine Influenza Virus
<400> 62
   Ile Ala Met Glu Leu Ile Arg Met Ile Lys Arg Gly Ile Asn Asp Arg
   1 5 10 15
<210> 63
   <211> 16
   <212> PRT
   <213> Artificial Sequence
<220>
   <223> Fragment of NP protein of Swine Influenza Virus
<400> 63
   Leu Ile Arg Met Ile Lys Arg Gly Ile Asn Asp Arg Asn Phe Trp Arg
   1 5 10 15
<210> 64
   <211> 16
   <212> PRT
   <213> Artificial Sequence
<220>
   <223> Fragment of NP protein of Swine Influenza Virus
<400> 64
   Ile Lys Arg Gly Ile Asn Asp Arg Asn Phe Trp Arg Gly Glu Asn Gly
   1 5 10 15
<210> 65
   <211> 16
   <212> PRT
   <213> Artificial Sequence
<220>
   <223> Fragment of NP protein of Swine Influenza Virus
<400> 65
   Ile Asn Asp Arg Asn Phe Trp Arg Gly Glu Asn Gly Arg Arg Thr Arg
   1 5 10 15
<210> 66
   <211> 16
   <212> PRT
   <213> Artificial Sequence
<220>
   <223> Fragment of NP protein of Swine Influenza Virus
<400> 66
   Asn Phe Trp Arg Gly Glu Asn Gly Arg Arg Thr Arg Ala Ala Tyr Glu
   1 5 10 15
<210> 67
   <211> 16
   <212> PRT
   <213> Artificial Sequence
<220>
   <223> Fragment of NP protein of Swine Influenza Virus
<400> 67
   Gly Glu Asn Gly Arg Arg Thr Arg Ala Ala Tyr Glu Arg Met Cys Asn
   1               5                   10                  15
<210> 68
   <211> 16
   <212> PRT
   <213> Artificial Sequence
<220>
   <223> Fragment of NP protein of Swine Influenza Virus
<400> 68
   Arg Arg Thr Arg Ala Ala Tyr Glu Arg Met Cys Asn Ile Leu Lys Gly
   1               5                   10                  15
<210> 69
   <211> 16
   <212> PRT
   <213> Artificial Sequence
<220>
   <223> Fragment of NP protein of Swine Influenza Virus
<400> 69
   Ala Ala Tyr Glu Arg Met Cys Asn Ile Leu Lys Gly Lys Phe Gln Thr
   1               5                   10                  15
<210> 70
   <211> 16
   <212> PRT
   <213> Artificial Sequence
<220>
   <223> Fragment of NP protein of Swine Influenza Virus
<400> 70
   Arg Met Cys Asn Ile Leu Lys Gly Lys Phe Gln Thr Ala Ala Gln Arg
   1               5                   10                  15
<210> 71
   <211> 16
   <212> PRT
   <213> Artificial Sequence
<220>
   <223> Fragment of NP protein of Swine Influenza Virus
<400> 71
   Ile Leu Lys Gly Lys Phe Gln Thr Ala Ala Gln Arg Ala Met Met Asp
   1 5 10 15
<210> 72
   <211> 16
   <212> PRT
   <213> Artificial Sequence
<220>
   <223> Fragment of NP protein of Swine Influenza Virus
<400> 72
   Lys Phe Gln Thr Ala Ala Gln Arg Ala Met Met Asp Gln Val Arg Glu
   1 5 10 15
<210> 73
   <211> 16
   <212> PRT
   <213> Artificial Sequence
<220>
   <223> Fragment of NP protein of Swine Influenza Virus
<400> 73
   Ala Ala Gln Arg Ala Met Met Asp Gln Val Arg Glu Ser Arg Asn Pro
   1 5 10 15
<210> 74
   <211> 16
   <212> PRT
   <213> Artificial Sequence
<220>
   <223> Fragment of NP protein of Swine Influenza Virus
<400> 74
   Ala Met Met Asp Gln Val Arg Glu Ser Arg Asn Pro Gly Asn Ala Glu
   1 5 10 15
<210> 75
   <211> 16
   <212> PRT
   <213> Artificial Sequence
<220>
   <223> Fragment of NP protein of Swine Influenza Virus
<400> 75
   Gln Val Arg Glu Ser Arg Asn Pro Gly Asn Ala Glu Ile Glu Asp Leu
   1 5 10 15
<210> 76
   <211> 16
   <212> PRT
   <213> Artificial Sequence
<220>
   <223> Fragment of NP protein of Swine Influenza Virus
<400> 76
   Ser Arg Asn Pro Gly Asn Ala Glu Ile Glu Asp Leu Ile Phe Leu Ala
   1 5 10 15
<210> 77
   <211> 16
   <212> PRT
   <213> Artificial Sequence
<220>
   <223> Fragment of NP protein of Swine Influenza Virus
<400> 77
   Gly Asn Ala Glu Ile Glu Asp Leu Ile Phe Leu Ala Arg Ser Ala Leu
   1 5 10 15
<210> 78
   <211> 16
   <212> PRT
   <213> Artificial Sequence
<220>
   <223> Fragment of NP protein of Swine Influenza Virus
<400> 78
   Ile Glu Asp Leu Ile Phe Leu Ala Arg Ser Ala Leu Val Leu Arg Gly
   1 5 10 15
<210> 79
   <211> 16
   <212> PRT
   <213> Artificial Sequence
<220>
   <223> Fragment of NP protein of Swine Influenza Virus
<400> 79
   Ile Phe Leu Ala Arg Ser Ala Leu Val Leu Arg Gly Ser Val Ala His
   1 5 10 15
<210> 80
   <211> 16
   <212> PRT
   <213> Artificial Sequence
<220>
   <223> Fragment of NP protein of Swine Influenza Virus
<400> 80
   Arg Ser Ala Leu Val Leu Arg Gly Ser Val Ala His Lys Ser Cys Leu
   1               5                   10                  15
<210> 81
   <211> 16
   <212> PRT
   <213> Artificial Sequence
<220>
   <223> Fragment of NP protein of Swine Influenza Virus
<400> 81
   Val Leu Arg Gly Ser Val Ala His Lys Ser Cys Leu Pro Ala Cys Val
   1               5                   10                  15
<210> 82
   <211> 16
   <212> PRT
   <213> Artificial Sequence
<220>
   <223> Fragment of NP protein of Swine Influenza Virus
<400> 82
   Ser Val Ala His Lys Ser Cys Leu Pro Ala Cys Val Tyr Gly Leu Ala
   1               5                   10                  15
<210> 83
   <211> 16
   <212> PRT
   <213> Artificial Sequence
<220>
   <223> Fragment of NP protein of Swine Influenza Virus
<400> 83
   Lys Ser Cys Leu Pro Ala Cys Val Tyr Gly Leu Ala Val Ala Ser Gly
   1               5                   10                  15
<210> 84
   <211> 16
   <212> PRT
   <213> Artificial Sequence
<220>
   <223> Fragment of NP protein of Swine Influenza Virus
<400> 84
   Pro Ala Cys Val Tyr Gly Leu Ala Val Ala Ser Gly His Asp Phe Glu
   1               5                   10                  15
<210> 85
   <211> 16
   <212> PRT
   <213> Artificial Sequence
<220>
   <223> Fragment of NP protein of Swine Influenza Virus
<400> 85
   Tyr Gly Leu Ala Val Ala Ser Gly His Asp Phe Glu Arg Glu Gly Tyr
   1               5                   10                  15
<210> 86
   <211> 16
   <212> PRT
   <213> Artificial Sequence
<220>
   <223> Fragment of NP protein of Swine Influenza Virus
<400> 86
   Val Ala Ser Gly His Asp Phe Glu Arg Glu Gly Tyr Ser Leu Val Gly
   1               5                   10                  15
<210> 87
   <211> 16
   <212> PRT
   <213> Artificial Sequence
<220>
   <223> Fragment of NP protein of Swine Influenza Virus
<400> 87
   His Asp Phe Glu Arg Glu Gly Tyr Ser Leu Val Gly Ile Asp Pro Phe
   1               5                   10                  15
<210> 88
   <211> 16
   <212> PRT
   <213> Artificial Sequence
<220>
   <223> Fragment of NP protein of Swine Influenza Virus
<400> 88
   Arg Glu Gly Tyr Ser Leu Val Gly Ile Asp Pro Phe Lys Leu Leu Gln
   1               5                   10                  15
<210> 89
   <211> 16
   <212> PRT
   <213> Artificial Sequence
<220>
   <223> Fragment of NP protein of Swine Influenza Virus
<400> 89
   Ser Leu Val Gly Ile Asp Pro Phe Lys Leu Leu Gln Asn Ser Gln Val
   1 5 10 15
<210> 90
   <211> 16
   <212> PRT
   <213> Artificial Sequence
<220>
   <223> Fragment of NP protein of Swine Influenza Virus
<400> 90
   Ile Asp Pro Phe Lys Leu Leu Gln Asn Ser Gln Val Phe Ser Leu Ile
   1 5 10 15
<210> 91
   <211> 16
   <212> PRT
   <213> Artificial Sequence
<220>
   <223> Fragment of NP protein of Swine Influenza Virus
<400> 91
   Lys Leu Leu Gln Asn Ser Gln Val Phe Ser Leu Ile Arg Pro Asn Glu
   1 5 10 15
<210> 92
   <211> 16
   <212> PRT
   <213> Artificial Sequence
<220>
   <223> Fragment of NP protein of Swine Influenza Virus
<400> 92
   Asn Ser Gln Val Phe Ser Leu Ile Arg Pro Asn Glu Asn Pro Ala His
   1 5 10 15
<210> 93
   <211> 16
   <212> PRT
   <213> Artificial Sequence
<220>
   <223> Fragment of NP protein of Swine Influenza Virus
<400> 93
   Phe Ser Leu Ile Arg Pro Asn Glu Asn Pro Ala His Lys Ser Gln Leu
   1 5 10 15
<210> 94
   <211> 16
   <212> PRT
   <213> Artificial Sequence
<220>
   <223> Fragment of NP protein of Swine Influenza Virus
<400> 94
   Arg Pro Asn Glu Asn Pro Ala His Lys Ser Gln Leu Val Trp Met Ala
   1               5                   10                  15
<210> 95
   <211> 16
   <212> PRT
   <213> Artificial Sequence
<220>
   <223> Fragment of NP protein of Swine Influenza Virus
<400> 95
   Asn Pro Ala His Lys Ser Gln Leu Val Trp Met Ala Cys His Ser Ala
   1               5                   10                  15
<210> 96
   <211> 16
   <212> PRT
   <213> Artificial Sequence
<220>
   <223> Fragment of NP protein of Swine Influenza Virus
<400> 96
   Lys Ser Gln Leu Val Trp Met Ala Cys His Ser Ala Ala Phe Glu Asp
   1               5                   10                  15
<210> 97
   <211> 16
   <212> PRT
   <213> Artificial Sequence
<220>
   <223> Fragment of NP protein of Swine Influenza Virus
<400> 97
   Val Trp Met Ala Cys His Ser Ala Ala Phe Glu Asp Leu Arg Val Ser
   1               5                   10                  15
<210> 98
   <211> 16
   <212> PRT
   <213> Artificial Sequence
<220>
   <223> Fragment of NP protein of Swine Influenza Virus
<400> 98
   Cys His Ser Ala Ala Phe Glu Asp Leu Arg Val Ser Ser Phe Ile Arg
   1 5 10 15
<210> 99
   <211> 16
   <212> PRT
   <213> Artificial Sequence
<220>
   <223> Fragment of NP protein of Swine Influenza Virus
<400> 99
   Ala Phe Glu Asp Leu Arg Val Ser Ser Phe Ile Arg Gly Lys Lys Val
   1 5 10 15
<210> 100
   <211> 16
   <212> PRT
   <213> Artificial Sequence
<220>
   <223> Fragment of NP protein of Swine Influenza Virus
<400> 100
   Leu Arg Val Ser Ser Phe Ile Arg Gly Lys Lys Val Ile Pro Arg Gly
   1 5 10 15
<210> 101
   <211> 16
   <212> PRT
   <213> Artificial Sequence
<220>
   <223> Fragment of NP protein of Swine Influenza Virus
<400> 101
   Ser Phe Ile Arg Gly Lys Lys Val Ile Pro Arg Gly Lys Leu Ser Thr
   1 5 10 15
<210> 102
   <211> 16
   <212> PRT
   <213> Artificial Sequence
<220>
   <223> Fragment of NP protein of Swine Influenza Virus
<400> 102
   Gly Lys Lys Val Ile Pro Arg Gly Lys Leu Ser Thr Arg Gly Val Gln
   1 5 10 15
<210> 103
   <211> 16
   <212> PRT
   <213> Artificial Sequence
<220>
   <223> Fragment of NP protein of Swine Influenza Virus
<400> 103
   Ile Pro Arg Gly Lys Leu Ser Thr Arg Gly Val Gln Ile Ala Ser Asn
   1               5                   10                  15
<210> 104
   <211> 16
   <212> PRT
   <213> Artificial Sequence
<220>
   <223> Fragment of NP protein of Swine Influenza Virus
<400> 104
   Lys Leu Ser Thr Arg Gly Val Gln Ile Ala Ser Asn Glu Asn Val Glu
   1               5                   10                  15
<210> 105
   <211> 16
   <212> PRT
   <213> Artificial Sequence
<220>
   <223> Fragment of NP protein of Swine Influenza Virus
<400> 105
   Arg Gly Val Gln Ile Ala Ser Asn Glu Asn Val Glu Ala Met Asp Ser
   1               5                   10                  15
<210> 106
   <211> 16
   <212> PRT
   <213> Artificial Sequence
<220>
   <223> Fragment of NP protein of Swine Influenza Virus
<400> 106
   Ile Ala Ser Asn Glu Asn Val Glu Ala Met Asp Ser Asn Thr Leu Glu
   1               5                   10                  15
<210> 107
   <211> 16
   <212> PRT
   <213> Artificial Sequence
<220>
   <223> Fragment of NP protein of Swine Influenza Virus
<400> 107
   Glu Asn Val Glu Ala Met Asp Ser Asn Thr Leu Glu Leu Arg Ser Arg
   1 5 10 15
<210> 108
   <211> 16
   <212> PRT
   <213> Artificial Sequence
<220>
   <223> Fragment of NP protein of Swine Influenza Virus
<400> 108
   Ala Met Asp Ser Asn Thr Leu Glu Leu Arg Ser Arg Tyr Trp Ala Ile
   1 5 10 15
<210> 109
   <211> 16
   <212> PRT
   <213> Artificial Sequence
<220>
   <223> Fragment of NP protein of Swine Influenza Virus
<400> 109
   Asn Thr Leu Glu Leu Arg Ser Arg Tyr Trp Ala Ile Arg Thr Arg Ser
   1 5 10 15
<210> 110
   <211> 16
   <212> PRT
   <213> Artificial Sequence
<220>
   <223> Fragment of NP protein of Swine Influenza Virus
<400> 110
   Leu Arg Ser Arg Tyr Trp Ala Ile Arg Thr Arg Ser Gly Gly Asn Thr
   1 5 10 15
<210> 111
   <211> 16
   <212> PRT
   <213> Artificial Sequence
<220>
   <223> Fragment of NP protein of Swine Influenza Virus
<400> 111
   Tyr Trp Ala Ile Arg Thr Arg Ser Gly Gly Asn Thr Asn Gln Gln Arg
   1 5 10 15
<210> 112
   <211> 16
   <212> PRT
   <213> Artificial Sequence
<220>
   <223> Fragment of NP protein of Swine Influenza Virus
<400> 112
   Arg Thr Arg Ser Gly Gly Asn Thr Asn Gln Gln Arg Ala Ser Ala Gly
   1               5                   10                  15
<210> 113
   <211> 16
   <212> PRT
   <213> Artificial Sequence
<220>
   <223> Fragment of NP protein of Swine Influenza Virus
<400> 113
   Gly Gly Asn Thr Asn Gln Gln Arg Ala Ser Ala Gly Gln Ile Ser Val
   1               5                   10                  15
<210> 114
   <211> 16
   <212> PRT
   <213> Artificial Sequence
<220>
   <223> Fragment of NP protein of Swine Influenza Virus
<400> 114
   Asn Gln Gln Arg Ala Ser Ala Gly Gln Ile Ser Val Gln Pro Thr Phe
   1               5                   10                  15
<210> 115
   <211> 16
   <212> PRT
   <213> Artificial Sequence
<220>
   <223> Fragment of NP protein of Swine Influenza Virus
<400> 115
   Ala Ser Ala Gly Gln Ile Ser Val Gln Pro Thr Phe Ser Val Gln Arg
   1               5                   10                  15
<210> 116
   <211> 16
   <212> PRT
   <213> Artificial Sequence
<220>
   <223> Fragment of NP protein of Swine Influenza Virus
<400> 116
   Gln Ile Ser Val Gln Pro Thr Phe Ser Val Gln Arg Asn Leu Pro Phe
   1 5 10 15
<210> 117
   <211> 16
   <212> PRT
   <213> Artificial Sequence
<220>
   <223> Fragment of NP protein of Swine Influenza Virus
<400> 117
   Gln Pro Thr Phe Ser Val Gln Arg Asn Leu Pro Phe Glu Arg Ala Thr
   1 5 10 15
<210> 118
   <211> 16
   <212> PRT
   <213> Artificial Sequence
<220>
   <223> Fragment of NP protein of Swine Influenza Virus
<400> 118
   Ser Val Gln Arg Asn Leu Pro Phe Glu Arg Ala Thr Ile Met Ala Ala
   1 5 10 15
<210> 119
   <211> 16
   <212> PRT
   <213> Artificial Sequence
<220>
   <223> Fragment of NP protein of Swine Influenza Virus
<400> 119
   Asn Leu Pro Phe Glu Arg Ala Thr Ile Met Ala Ala Phe Ser Gly Asn
   1 5 10 15
<210> 120
   <211> 16
   <212> PRT
   <213> Artificial Sequence
<220>
   <223> Fragment of NP protein of Swine Influenza Virus
<400> 120
   Glu Arg Ala Thr Ile Met Ala Ala Phe Ser Gly Asn Asn Glu Gly Arg
   1 5 10 15
<210> 121
   <211> 16
   <212> PRT
   <213> Artificial Sequence
<220>
   <223> Fragment of NP protein of Swine Influenza Virus
<400> 121
   Ile Met Ala Ala Phe Ser Gly Asn Asn Glu Gly Arg Thr Ser Asp Met
   1               5                   10                  15
<210> 122
   <211> 16
   <212> PRT
   <213> Artificial Sequence
<220>
   <223> Fragment of NP protein of Swine Influenza Virus
<400> 122
   Phe Ser Gly Asn Asn Glu Gly Arg Thr Ser Asp Met Arg Thr Glu Val
   1               5                   10                  15
<210> 123
   <211> 16
   <212> PRT
   <213> Artificial Sequence
<220>
   <223> Fragment of NP protein of Swine Influenza Virus
<400> 123
   Asn Glu Gly Arg Thr Ser Asp Met Arg Thr Glu Val Ile Arg Met Met
   1               5                   10                  15
<210> 124
   <211> 16
   <212> PRT
   <213> Artificial Sequence
<220>
   <223> Fragment of NP protein of Swine Influenza Virus
<400> 124
   Thr Ser Asp Met Arg Thr Glu Val Ile Arg Met Met Glu Ser Ala Lys
   1               5                   10                  15
<210> 125
   <211> 16
   <212> PRT
   <213> Artificial Sequence
<220>
   <223> Fragment of NP protein of Swine Influenza Virus
<400> 125
   Arg Thr Glu Val Ile Arg Met Met Glu Ser Ala Lys Pro Glu Asp Leu
   1               5                   10                  15
<210> 126
   <211> 16
   <212> PRT
   <213> Artificial Sequence
<220>
   <223> Fragment of NP protein of Swine Influenza Virus
<400> 126
   Ile Arg Met Met Glu Ser Ala Lys Pro Glu Asp Leu Ser Phe Gln Gly
   1               5                   10                  15
<210> 127
   <211> 16
   <212> PRT
   <213> Artificial Sequence
<220>
   <223> Fragment of NP protein of Swine Influenza Virus
<400> 127
   Glu Ser Ala Lys Pro Glu Asp Leu Ser Phe Gln Gly Arg Gly Val Phe
   1               5                   10                  15
<210> 128
   <211> 16
   <212> PRT
   <213> Artificial Sequence
<220>
   <223> Fragment of NP protein of Swine Influenza Virus
<400> 128
   Pro Glu Asp Leu Ser Phe Gln Gly Arg Gly Val Phe Glu Leu Ser Asp
   1               5                   10                  15
<210> 129
   <211> 16
   <212> PRT
   <213> Artificial Sequence
<220>
   <223> Fragment of NP protein of Swine Influenza Virus
<400> 129
   Ser Phe Gln Gly Arg Gly Val Phe Glu Leu Ser Asp Glu Lys Ala Thr
   1               5                   10                  15
<210> 130
   <211> 16
   <212> PRT
   <213> Artificial Sequence
<220>
   <223> Fragment of NP protein of Swine Influenza Virus
<400> 130
   Arg Gly Val Phe Glu Leu Ser Asp Glu Lys Ala Thr Ser Pro Ile Val
   1               5                   10                  15
<210> 131
   <211> 16
   <212> PRT
   <213> Artificial Sequence
<220>
   <223> Fragment of NP protein of Swine Influenza Virus
<400> 131
   Glu Leu Ser Asp Glu Lys Ala Thr Ser Pro Ile Val Pro Ser Phe Asp
   1               5                   10                  15
<210> 132
   <211> 16
   <212> PRT
   <213> Artificial Sequence
<220>
   <223> Fragment of NP protein of Swine Influenza Virus
<400> 132
   Glu Lys Ala Thr Ser Pro Ile Val Pro Ser Phe Asp Met Ser Asn Glu
   1               5                   10                  15
<210> 133
   <211> 16
   <212> PRT
   <213> Artificial Sequence
<220>
   <223> Fragment of NP protein of Swine Influenza Virus
<400> 133
   Ser Pro Ile Val Pro Ser Phe Asp Met Ser Asn Glu Gly Ser Tyr Phe
   1               5                   10                  15
<210> 134
   <211> 16
   <212> PRT
   <213> Artificial Sequence
<220>
   <223> Fragment of NP protein of Swine Influenza Virus
<400> 134
   Pro Ser Phe Asp Met Ser Asn Glu Gly Ser Tyr Phe Phe Gly Asp Asn
   1 5 10 15
<210> 135
   <211> 16
   <212> PRT
   <213> Artificial Sequence
<220>
   <223> Fragment of NP protein of Swine Influenza Virus
<400> 135
   Met Ser Asn Glu Gly Ser Tyr Phe Phe Gly Asp Asn Ala Glu Glu Tyr
   1 5 10 15
<210> 136
   <211> 14
   <212> PRT
   <213> Artificial Sequence
<220>
   <223> Fragment of NP protein of Swine Influenza Virus
<400> 136
   Gly Ser Tyr Phe Phe Gly Asp Asn Ala Glu Glu Tyr Asp Ser
   1 5 10

## Description

### FIELD OF THE INVENTION

This invention is in the field of vaccine adjuvants.

### BACKGROUND

In the area of vaccinology, antigens are introduced into a host in a manner so as to stimulate an immune response to the antigen and therefore to the potential pathogen. The induction of an immune response depends on many factors among which are believed to be the chemical composition, characteristics and configuration of the antigen, the health and immune competence of the host, and the manner of delivery and administration of the antigen.

An immune response has many facets, some of which are exhibited by the cells of the immune system, (*e.g*., Dendritic cells, B-lymphocytes, T-lymphocytes, macrophages, and plasma cells). Cells of the immune system participate in the immune response through interaction with antigens or other cells of the immune system, the release of cytokines and reactivity to those cytokines. Adaptive (acquired) immune response is conveniently (but arbitrarily) divided Into two main categories--humoral and cell-mediated. The humoral component of the immune response includes production of antibodies specific for the antigen. The cell-mediated component includes the generation of delayed-type hypersensitivity and cytotoxic effector T-cells specific to the antigen.

Adjuvants are substances used to potentiate an immune response when used in conjunction with the antigen. The use of an adjuvant in a vaccination protocol may, for example, elicit an immune response that is faster or greater than would be elicited with antigen alone. In addition, adjuvants may be used to direct the immune response to specific immunological pathways and to serve as a delivery vehicle for the antigen.

Liposomes and liposomal formulations are examples of adjuvants. Typically liposomes can be loaded with the antigen(s) and/or other immunomodulatory compounds, or the liposomes themselves may serve as standalone adjuvants. The antigens and/or other immunostimulatory compounds may be encapsulated in the interior of the liposome, and/or they can be attached to the liposome or incorporated Into the lipid bilayer.

The factors influencing the suitability of a given liposome as a delivery vehicle in a given system presentation remain unclear. Thus there is still a need for delivery vehicles, which provide an improved efficacy. Such an improved delivery is particular for the administration of molecules which stimulate and/or elicit an immune response, for example, antigens and immunomodulators. Australian Patent No. AU 750379 B2 discloses a pharmaceutical composition comprising an immunogenic agent derived from Helicobacter and at least one compound capable of promoting the induction of a T helper 1 (Thl) type immune response against Helicobacter selected from: (i) saponins purified from an extract of Quillaja saponaria; (ii) cationic lipids or a salt of the latter; on condition that these lipids are not provided in the form of liposomes when the said composition contains no saponin or glycolipopeptide of formula (I); and (iii) glycolipopeptides of formula (I).

### SUMMARY OF INVENTION

The instant invention is directed to adjuvants for enhancing the performance of a vaccine.

Any references in the description to methods of treatment refer to the compounds, pharmaceutical compositions and medicaments of the present invention for use in a method for treatment of the human or animal body by therapy. The invention provides an essentially saponin-free liposome comprising an external lipid bilayer membrane and an internal compartment,the external membrane comprising: a) a quaternary ammonium compound composed of four alkyl chains, two of which are C10-C20 alkyls and the remaining two are C1-C4 alkyls;b) a sterol selected from the group consisting of p-sitosterol, stigmasterol, ergosterol, ergocalciferol, and cholesterol;c) a phospholipid; and d) a glycolipid of Formula I: 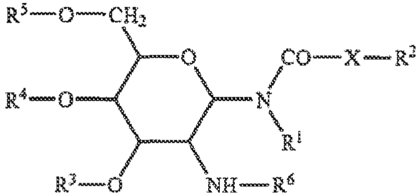 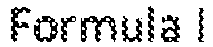 wherein, R¹ is hydrogen, or a saturated alkyl radical having up to 20 carbon atoms; X is -CH₂-, -O- or -NH-; R² is hydrogen, or a saturated or unsaturated alkyl radical having up to 20 carbon atoms; R³, R⁴, and R⁵ are independently hydrogen, -SO₄²⁻, -PO₄²⁻, -COC_{1- 10} alkyl; R⁶ is L-alanyl, L-alpha-aminobutyl, L-arginyl, L-asparginyl, L-aspartyl, L-cysteinyl, L-glutamyl, L-glycyl, L-histidyl, L-hydroxyprolyl, L-isoleucyl, L-leucyl, L-lysyl, L-methionyl, L-ornithlnyl, L-phenyalany, L-prolyl, L-seryl, L-threonyl, L-tyrosyl, L-tryptophanyl, and L-valyl or their D-isomers.

In certain embodiments, the quaternary ammonium compound is DDA, the sterol is cholesterol, the phospholipid is lecithin, and the glycolipid is N-(2-Deoxy-2-L-leucylamino-β-D-glucopyranosyl)-N-octadecyldodecanoylamide or an acetate thereof.

The liposome is essentially saponin-free.

In certain embodiments, the liposome further comprises an immunostimulatory oligonucleotide selected from the group consisting of an immunostimulatory ribonucleotide, a CpG oligodeoxyribonucleotide, and a combination thereof. In some embodiments, the liposome is free of CpG oligodeoxyribonucleotide.

In some embodiments, the immunostimulatory oligonucleotide is incorporated within the internal compartment of the liposome. In other embodiments, the immunostimulatory oligonucleotide is associated with the outer surface of the liposome.

In some embodiments, said immunostimulatory oligonucleotide comprises any one of SEQ ID NOs 1-14.

In certain aspects, the invention provides an adjuvant formulation comprising the liposomes as described herein.

In certain embodiments, the adjuvant formulation is essentially saponin-free. In certain embodiments, the adjuvant formulation is essentially free of CpG oligodeoxyribonucleotide.

In certain aspects, the invention provides a vaccine composition comprising an effective amount of an antigenic component and an adjuvant formulation as described herein.

In certain embodiments, the vaccine composition is essentially saponin-free.

In certain embodiments, the vaccine composition is essentially free of CpG. In certain embodiments, the antigenic component of the essentially CpG-free vaccine composition contains a (-)ssRNA virus.

In certain embodiments, the (-)ssRNA virus is an influenza virus. In some embodiments, the influenza virus is a Swine influenza Virus.

In certain embodiments, the antigenic component is incorporated within the internal compartment of the liposome.

The antigenic component, in selected embodiments suitable for cattle, may include BVDV-1 and/or BVDV-2 inactivated viruses (and BHV-1). In other embodiments, particularly suitable for poultry animals, the antigenic component includes profilin.

### DETAILED DESCRIPTION

### Definitions:

The terms 'about' or 'approximately,' when used in connection with a measurable numerical variable, refer to the indicated value of the variable and to all values of the variable that are within the experimental error of the indicated value (*e.g*., within the 95% confidence interval for the mean) or within 10 percent of the indicated value, whichever is greater, unless 'about' is used in reference to time intervals in weeks where "about 3 weeks," is 17 to 25 days, and about 2 to about 4 weeks is 10 to 40 days.

The term 'accompanying fever' refers to rise in temperature of the vaccinated animal within one day of vaccination. In case of bovines, the term refers to rectal temperature over 103.5 °F.

The term 'antigen' in combination with the species refers to pathogens causing infectious disease in said species, or to the components of these pathogens. Thus, for example, 'bovine antigens' refer to pathogens capable of causing infections disease in bovines or to the components of these pathogens.

The term 'consisting essentially of and the like as applied to the liposomes and the adjuvant formulations of the instant invention refers to compositions which do not contain additional adjuvanting or immunomodulating agents in the amounts at which said agent exert measurable adjuvanting or immunomodulating effects.

The terms "essentially saponin-free', 'substantially saponin-free' and the like refer to a composition that does not contain saponin in the amounts at which saponin exerts measurable adjuvanting or immunomodulating effects. In certain embodiments, essentially saponin-free compositions contain saponin in the amount insufficient to cause systemic immune response, such as fever. In certain embodiments, essentially saponin-free compositions contain no saponin or contain saponin at or below the limit of detection.

Similarly, the terms 'essentially free of CpG deoxyribonucleotide', 'substantially free of CpG deoxyribonucleotide' and the like refer to a composition which does not contain CpG deoxyribonucleotide in the amounts at which the CpG deoxyribonucleotide exerts measurable adjuvanting or immunomodulating effects. In certain embodiments, compositions essentially free of CpG deoxyribonucleotide contain no CpG deoxyribonucleotide or contain CpG deoxyribonucleotide at or below the limit of detection. The terms 'essentially free of CpG deoxyribonucleotide', 'substantially free of CpG deoxyribonucleotide' and the like specifically exclude the vaccines where the CpG deoxyribonucleotide is naturally present in the antigen.

The term 'immunostimulatory molecule' refers to a molecule that enhances an immune response.

The term 'liposome' refers to a microscopic spherical particle formed by a lipid bilayer enclosing an aqueous compartment.

The term 'parenteral administration' refers to the introduction of a substance, such as a vaccine, into a subject's body through or by way of a route that does not include the digestive tract. Parenteral administration includes subcutaneous, intramuscular, transcutaneous, intradermal, intraperitoneal, intraocular, and intravenous administration.

The terms 'therapeutically effective amount' and 'effective amount' refer to an amount of an antigen or vaccine that would induce an immune response in a subject receiving the antigen or vaccine which is adequate to prevent or reduce signs or symptoms of disease, including adverse health effects or complications thereof, caused by infection with a pathogen, such as a virus or a bacterium. Humoral immunity or cell-mediated immunity or both humoral and cell-mediated immunity may be induced. The immunogenicity and efficacy of a vaccine in an animal may be evaluated, *e.g*., indirectly through measurement of antibody titers, lymphocyte proliferation assays, IFN gamma ELISPOT assays, cytotoxic T cell assays or directly through monitoring signs and symptoms after challenge with wild type strain. The protective immunity conferred by a vaccine can be evaluated by measuring, *e.g*., reduction in clinical signs such as mortality, morbidity, fever, viremia, impact on clinical pathology, overall physical condition, and overall health and performance of the subject. The amount of a vaccine that is therapeutically effective may vary depending on the particular adjuvant used, the particular antigen used, or the condition of the subject, and can be determined by one skilled in the art.

The invention provides, in part, liposomes containing an internal compartment and an external membrane. Liposomes may have average particle size between 50 and 500 nm. In certain non-limiting embodiments, the average particle size of the liposomes of is 100-500 nm, or 150-450 nm, or 150-250 nm, or 300-400 nm, or 250-300 nm. In certain embodiments, the membrane comprises a quaternary amine compound, a phospholipid, a sterol, and a glycolipid. In certain embodiments, the liposome is essentially free of saponin.

In certain embodiments, the external membrane consists essentially or consists of the quaternary amine compound, the phospholipid, the sterol, and the glycolipid. In other embodiments, the external compartment of the liposome does not contain any immunostimulatory oligonucleotides and/or other immunomodulatory compounds. Thus, in such embodiments, the liposome consists essentially of, or consists of the internal compartment consisting essentially of or consisting of an immunologically inert aqueous vehicle, said internal compartment surrounded by the external membrane which consists essentially of, or consists of the quaternary amine compound, the phospholipid, the sterol, and the glycolipid.

Quaternary amine compounds are ammonium based compounds with four hydrocarbon groups. The quaternary amine compounds are composed of four alkyl chains, two of which are C10-C20 alkyls and the remaining two are C1-C4 alkyls. In certain embodiments, the quaternary amine is dimethyldioctadecylammonium (DDA) bromide, chloride or another pharmaceutically acceptable counter ion.

Sterols share a common chemical core, which is a steroid ring structure[s], having a hydroxyl (OH) group, usually attached to carbon-3. The hydrocarbon chain of the fatty-acid substituent varies in length, usually from 16 to 20 carbon atoms, and can be saturated or unsaturated. Sterols commonly contain one or more double bonds in the ring structure and also a variety of substituents attached to the rings. Sterols and their fatty-acid esters are essentially water-insoluble. In view of these chemical similarities, it is thus likely that the sterols sharing this chemical core would have similar properties when used in the vaccine compositions of the instant invention. Sterols are well known in the art and can be purchased commercially. For example cholesterol is disclosed in the Merck Index, 12th Ed., p. 369. Suitable sterols include, without limitations, β-sitosterol, stigmasterol, ergosterol, ergocalciferol, and cholesterol.

Suitable glycolipids are generally those which activate the Th2 response. The glycolipids include, without limitations, those encompassed by Formula I and that are generally described in US Patent Publication 20070196384 (Ramasamy et al).

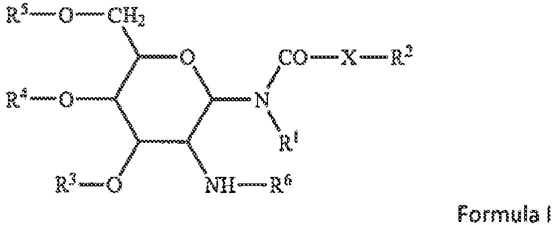

In the structure of Formula I, R¹ is hydrogen, or a saturated alkyl radical having up to 20 carbon atoms; X is -CH₂-, -O- or -NH-; R² is hydrogen, or a saturated or unsaturated alkyl radical having up to 20 carbon atoms; R³, R⁴, and R⁵ are independently hydrogen, -SO₄²⁻, -PO₄²⁻, -COC₁₋₁₀ alkyl; R⁶ is L-alanyl, L-alpha-aminobutyl, L-arginyl, L-asparginyl, L-aspartyl, L-cysteinyl, L-glutamyl, L-glycyl, L-histidyl, L-hydroxyprolyl, L-isoleucyl, L-leucyl, L-lysyl, L-methionyl, L-ornithinyl, L-phenyalany, L-prolyl, L-seryl, L-threonyl, L-tyrosyl, L-tryptophanyl, and L-valyl or their D-isomers.

Examples of a glycolipid are, without limitation, N-(2-Deoxy-2-L-leucylamino-β-D-glucopyranosyl)-N-octadecyldodecanoylamide (BayR®1005, or R1005) or a salt (*e.g.*, an acetate) thereof.

Lecithin can be obtained as a mixture of phosphatides and triglycerides by water-washing crude vegetable oils, and separating and drying the resulting hydrated gums. A refined product can be obtained by fractionating the mixture for acetone insoluble phospholipids and glycolipids remaining after removal of the triglycerides and vegetable oil by acetone washing. Alternatively, lecithin can be obtained from various commercial sources.

Other suitable phospholipids include phosphatidylcholine, phosphatidylglycerol, phosphatidylinositol, phosphatidylserine, acylphosphatidylethanolamine, diphosphatidiglcerol, lysophosphatidylethanolamine, lysophosphatidylcholine, phosphatidic acid, cardiolipin, and phosphatidylethanolamine. The phospholipids may be isolated from natural sources or conventionally synthesized.

The liposomes as described herein allow for flexible ratios of the elements of the external membrane. In certain embodiments, the weight ratios of the quaternary ammonium compound: the sterol: the phospholipid: the glycolipid are 1: 0.75-1.25: 1.5-2.5: 1.5-2.5, respectively. In certain embodiments, the weight ratios of the quaternary ammonium compound: the sterol: the phospholipid: the glycolipid are 1: 1: 2: 2.

In other embodiments, the total weight of the quaternary ammonium compound and the sterol is about the half (*e.g*., 40%, 45%, 50%, 55%, 60%) of the total weight of the glycolipid and the phospholipid, provided that the quaternary ammonium compound comprises at least about 5% w/w of the total weight of these four compounds (the quaternary ammonium compound, the sterol, the phospholipid, and the glycolipid), and the glycolipid is at least about 20% w/w of the total weight of these four compounds.

In certain embodiments, the total weight of the quaternary ammonium compound and the sterol is about 10-40% (*e.g*., about 10%, about 15%, about 20%, about 25%, about 30%, about 33.3%, about 35%, about 40%) of the total weight of the glycolipid and the phospholipid, provided that the quaternary ammonium compound comprises at least about 5% w/w of the total weight of these four compounds (the quaternary ammonium compound, the sterol, the phospholipid, and the glycolipid), and the glycolipid is at least about 20% w/w of the total weight of these four compounds.

In certain embodiments, immunologically effective amount of the liposomes of the instant invention may be administered as an adjuvant. In some embodiments, the invention provides a vaccine combination comprising the immunologically effective amount of the adjuvant composition, and the antigenic component, as further described below.

The weight of the client species ultimately dictates the dose of the adjuvant composition of the instant invention.

In certain embodiments, suitable for cattle, horses, and adult pigs, one dose contains the equivalent of 1000-3000 µg of external membrane component (*i.e*., the total weight of the quaternary ammonium compound, the sterol, the phospholipid, and the glycolipid), or the equivalent of 1000-2000 µg, or the equivalent of 1000-1500 µg, or the equivalent of 1300-1800 µg, or the equivalent of 1500-2000 µg.

The weight of the liposome composition may not be equal to the weight of the membrane component due to the presence of the internal compartment which may contain the immunostimulatory oligonucleotide, the antigen component, other immunomodulators, etc. The use of equivalents to the liposomal membrane component allows for the uniform dosing. The dosing regimen recited therein ensures that the cattle animal receives at least 200 µg of the glycolipid and about 50 µg of the quaternary ammonium compound.

In certain embodiments suitable for sheep and goats, one dose contains the equivalent of 300-1000 µg of external membrane component, *e.g*., the equivalents of 300-500 µg, or the equivalents of 400-500 µg, or the equivalents of 400-1000 µg, or the equivalents of 500-1000 µg, or the equivalents of 600-1000 µg, or the equivalents of 600-800 µg.

In certain embodiments suitable for piglets, dogs, and cats, one dose contains the equivalent of 100-400 µg of external membrane component, or the equivalent of 100-200 µg, or the equivalent of 100-150 µg, or the equivalent of 130-180 µg, or the equivalent of 150-200 µg.

In certain embodiments suitable for poultry, one dose contains the equivalent of 50-200 µg of external membrane component, or the equivalent of 50-100 µg, or the equivalent of 50-75 µg, or the equivalent of 65-90 µg, or the equivalent of 75-100 µg, or the equivalent of 75-150 µg.

The internal compound of the liposome may contain antigens or other immunomodulatory molecules. In certain embodiments, such immunomodulatory molecules suitable for the internal compartment include, without limitation, antigen extracts, subunits, synthetics, whole cell or virus.

In certain embodiments, active pharmaceuticals may be packaged inside a liposome.

Immunomodulators that could be packaged also include, without limitations, rmLT, MPLA, Alpha-Gal-Cer. Cholera toxin, LPS, lipoteichoic acids, poly I:C, flagellin, zymosan, chitin and modified chitin forms, beta-glucans, avridine, inulin and modified inulin forms, ethylene malic anhydrtes, pluronics like L121 and L141, CD40agonist, TLR5 agonist as well as any TLR agonist, GM-CSF.

In certain embodiments, liposomes may carry various molecules that could be used as markers, including without limitations, OspA, OspC, pertactin and others.

In certain embodiments, the adjuvant composition of the instant invention further comprises immunostimulatory oligonucleotides, such as, for example, CpG oligodeoxyribonucleotides or immunostimulatory oligoribonucleotides (ORNs), or chimeras thereof. Suitable non-limiting examples of CpG oligodeoxyribonucleotides are illustrated in SEQ ID NOs 1-10, suitable non-limiting examples of ORNs are provided in SEQ ID NOs 11-13, and a suitable non-limiting example of a chimeric immunostimulatory oligonucleotide is provided in SEQ ID NO: 14.

These immunostimulatory oligonucleotides are, in some embodiments, present in the internal compartment of the liposome.

In certain embodiments, the Immunomodulatory oligonucleotides are associated with the outer surface of the liposome. The association may be due to hydrogen bonds, electrostatic bonds, lipophilic bonds, Van der Waals forces, and the like.

In certain embodiments, the negatively charged immunostimulatory oligonucleotide is associated with the outer surface of the liposome due to interaction with the positively charged quaternary nitrogen atom in the quaternary ammonium compound.

CpG oligodeoxyribonucleotides (also referred to as CpG deoxyribonucleotides or CpG ODN) are a recently described class of pharmacotherapeutic agents that are characterized by the presence of an unmethylated CG dinucleotide in specific base-sequence contexts (CpG motif). (Hansel TT, Barnes PJ (eds): New Drugs for Asthma, Allergy and COPD. Prog Respir Res. Basel, Karger, 2001, vol 31, pp 229-232, which is incorporated herein by reference). These CpG motifs are not seen in eukaryotic DNA, in which CG dinucleotides are suppressed and, when present, usually methylated, but are present in bacterial DNA to which they confer immunostimulatory properties.

In certain embodiments, the adjuvants of the instant invention utilize a so-called P-class immunostimulatory oligonucleotide, more preferably, modified P- class immunostimulatory oligonucleotides, even more preferably, E-modified P-class oligonucleotides. P-class Immunostimulatory oligonucleotides are CpG oligodeoxyribonucleotide characterized by the presence of palindromes, generally 6-20 nucleotides long. The P-Class oligonucleotides have the ability to spontaneously self-assemble into concatamers either in vitro and/or in vivo. These oligonucleotides are, in a strict sense, single-stranded, but the presence of palindromes allows for formation of concatamers or possibly stem-and-loop structures. The overall length of P-class immunostimulatory oligonucleotides is between 19 and 100 nucleotides, *e.g.*, 19-30 nucleotides, 30-40 nucleotides, 40-50 nucleotides, 50-60 nucleotides, 60-70 nucleotides, 70-80 nucleotides, 80-90 nucleotides, 90-100 nucleotides.

In one aspect of the invention the immunostimulatory oligonucleotide contains a 5' TLR activation domain and at least two palindromic regions, one palindromic region being a 5' palindromic region of at least 6 nucleotides in length and connected to a 3' palindromic region of at least 8 nucleotides in length either directly or through a spacer.

The P-class immunostimulatory oligonucleotides may be modified according to techniques known in the art. For example, J-modification refers to iodo-modified nucleotides. E-modification refers to ethyl-modified nucleotide(s). Thus, E-modified P-class immunostimulatory oligonucleotides are P-class immunostimulatory oligonucleotides, wherein at least one nucleotide (preferably 5' nucleotide) is ethylated. Additional modifications include attachment of 6-nitro-benzimldazol, O-Methylation, modification with proynyl-dU, inosine modification, 2-bromovinyl attachment (preferably to uridine).

The P-class immunostimulatory oligonucleotides may also contain a modified internucleotide linkage including, without limitations, phosphodiester linkages and phosphorothioate linkages. The oligonucleotides of the instant invention may be synthesized or obtained from commercial sources.

P-Class oligonucleotides and modified P-class oligonucleotides are further disclosed in published PCT application no. WO2008/068638, published on Jun. 12, 2008. Suitable non-limiting examples of modified P-class immunostimulatory oligonucleotides are provided below (In SEQ ID NOs 1-10, "*" refers to a phosphorothioate bond and "_" refers to a phosphodiester bond). In SEQ ID NOs 11-14, all bonds are either phosphodiester or phosphorothioate bonds.
SEQ ID NO: 1 5' T*C_G*T*C_G*A*C_G*A*T*C_G*G*C*G*C_G*C*G*C*C*G 3'
SEQ ID NO: 2 5' T*C_G*A*C*G*T*C*G*A*T*C*G*G*C*G*C*G*C*G*C*C*G 3'
SEQ ID NO: 3 5' T*C*G*A*C*G*T*C*G*A*T*C*G*G*C*G*C*G*C*G*C*C*G*T 3'
SEQ ID NO: 4 5' JU*C_G*A*C*G*T*C*G*A*T*C*G*G*C*G*C*G*C*G*C*C*G 3'
SEQ ID NO: 5 5' JU*C_G*A*C*G*T*C*G*A*T*C*G*G*C*G*C*G*C*G*C*C*G*T 3'
SEQ ID NO: 6 5' JU*C*G*A*C*G*T*C*G*A*T*C*G*G*C*G*C*G*C*G*C*C*G*T 3'
SEQ ID NO: 7 5' EU*C_G*A*C*G*T*C*G*A*T*C*G*G*C*G*C*G*C*G*C*C*G 3'
SEQ ID NO: 8 5' JU*C_G*T*C*G*A*C*G*A*T*C*G*G*C*G*G*C*C*G*C*C*G*T 3'
SEQ ID NO: 9 5' JU*C*G*T*C*G*A*C*G*A*T*C*G*G*C*G*G*C*C*G*C*C*G*T 3'
SEQ ID NO: 10 5' T*C_G*T*C_G'A*C_G*A*T*C_G*G*C*G*C_G*C*G*C*C*G 3'
SEQ ID NO: 11 5'-UUGUUGUUGUUGUUGUUGUU-3'
SEQ ID NO: 12 5'-UUAUUAUUAUUAUUAUUAUU-3'
SEQ ID NO: 13 5'-AAACGCUCAGCCAAAGCAG-3'
SEQ ID NO: 14 5'-dTdCdGdTdCdGdTdTdTdTrGrUrUrGrUrGrUdTdTdTdT-3'

The dose of the immunostimulatory oligonucleotide for use in the adjuvant compositions ultimately depends upon the intended species.

For example, in certain embodiments suitable for cattle, sheep or adult swine, one dose of the adjuvant composition of the instant invention would comprise between about 50 and 400 µg (*e.g.*, 50-300, or 100-250 µg, or about 50 to about 100 µg for adult pigs and about 100 to about 250 µg for cattle) of the immunostimulatory oligonucleotide.

In certain embodiments suitable for companion animals or piglets, one dose of the adjuvant composition of the instant invention would comprise between about 5 and 100 µg (e.g., 10-80 µg, or 20-50 µg) of the immunostimulatory oligonucleotide.

In certain embodiments suitable for poultry, one dose of the adjuvant composition of the instant invention would between about 0.1 and about 5 µg (e.g., 0.5-3 µg, or 0.9-1.1 µg) of immunostimulatory oligonucleotide.

Methods of making liposomes are well known in the art. Briefly, the components of the liposome are dissolved and mixed in an organic solvent, e.g., methylene chloride, and then the solvent is removed by drying to yield a film. The film is later rehydrated using an aqueous media (e.g., water or a buffer) which optionally contains compounds which are to be incorporated within the internal compartment of the liposomes. In different embodiments, the compounds may include the immunostimulatory oligonucleotides, other immunomodulators, and/or the antigen component.

The step of rehydration is followed by sonication and/or extrusion to reduce the size of the vesicles formed during the rehydration step.

There are two main sonication techniques: probe/tip sonication and bath sonication. Probe/tip sonication has a high energy input which causes significant heat generation, therefore necessitating the use of an ice bath to maintain temperature of the liposomal dispersion in order to prevent lipid degradation. Alternatively, ultrasonic energy can be indirectly imparted to the liposome suspension using a bath sonicator, where temperature is easier to control but energy loss is comparatively high. Sonication generally yields small vesicles (^{∼}10 nm) which spontaneously fuse over time to relieve the stress of high membrane curvature.

The extrusion method involves passing the liposome suspension through a membrane with defined pore size. This method is advantageous because the defined pore size encourages homogeneity of particle size within the liposome population, though extrusion below lipid transition temperature can be difficult owing to membrane rigidity. Liposome suspensions are often extruded multiple times to achieve low polydispersity in the final product.

It may be desirable to prepare storage-stable preparation of liposomes. In certain embodiments, such storage-stable preparation of liposomes is created by freeze-drying. Briefly, the dry film described above, is rehydrated In an aqueous buffer containing a cryoprotectant and lyoprotectant such as sucrose, trehalose, or a combination thereof. In other embodiments, the cryoprotectant and lyoprotectant are added after the rehydration step. The rehydrated preparation is then lyophilized using techniques well known in the art. The resulting lyophilized preparation is storage-stable. At the desired time, it can be rehydrated with suitable buffer.

Additional immunomodulators, including, without limitations, the immunostimulatory oligonucleotides and the antigen(s) may be added either before the freeze-drying or at the time of final preparation.

In certain embodiments, the antigens are admixed with the liposomal formulation after the liposomes of the instant invention are reconstituted. In other embodiments, the liposomes, the additional immunomodulators, and the antigenic component are prepared and dried together.

In certain embodiments, additional immunostimulatory compounds are present in the compositions of the instant invention. Such additional immunostimulatory compounds may be present within the internal compartment of the liposomes, and/or associated with the outer surface of the liposomes, and/or independently of the liposomes, in the adjuvant compositions of the instant invention.

Suitable non-limiting examples of such additional immunostimulatory compounds include, but not limited to several adjuvant classes such as mineral salts, *e.g.*, Alum, aluminum hydroxide, aluminum phosphate and calcium phosphate; surface-active agents and microparticles, *e.g.*, nonionic block polymer surfactants, virosomes, saponins (*e.g.*, Quil A, QS-21 and GPI-0100), proteosomes, immune stimulating complexes, cochleates, pyridine, vitamin A, vitamin E; bacterial products such as the RIBI adjuvant system (Ribi Inc.), cell wail skeleton of Mycobacterium phlei (Detox®), muramyl dipeptides (MDP) and tripeptides (MTP), monophosphoryl lipid A (MPLA), Bacillus Calmete-Guerin (BCG), heat labile E. coli enterotoxins, cholera toxin, trehalose dimycolate, cytokines and hormones, *e.g.*, interleukins (IL-2, IL-2, IL-6, IL-12, IL-15, IL-18), granulocyte-macrophage colony stimulating factor, dehydroepiandrosterone, 1,25-dihydroxy vitamin D₃; polyanions, *e.g.*, dextran; polyacrylics (*e.g*., polymethylmethacrylate, CARBOPOL®934P); carriers *e.g.*, tetanus toxoid, diptheria toxoid, cholera toxin B subunit, mutant heat labile enterotoxin of enterotoxigenic E. coli (rmLT), heat shock proteins; oil-in-water emulsions *e.g*., AMPHIGEN® (Hydronics, USA); polycationic carriers (*e.g.*, DEAE Dextran or QAE Dextran), and water-in-oil emulsions such as, *e.g.*, Freund's complete and incomplete adjuvants.

Other suitable Immunomodulators include Alpha-Gal-Cer. LPS, lipoteichoic acids, poly I:C, flagellin, zymosan, chitin and modified chitin forms, beta-glucans, avridine, inulin and modified inulin forms, ethylene malic anhydries, pluronics like L121 and L141, CD40 agonist, TLR5 agonist as well as any TLR agonist.

### Antigens and Diseases

In certain embodiments, the liposomal adjuvant composition of the instant invention may be combined with an antigenic component thus forming the vaccine composition of the instant invention. The antigenic component of the vaccines of the instant invention may be present within the internal compartment of the liposomes, and/or associated with the outer surface of the liposomes, and/or independently of the liposomes.

In certain embodiments, the vaccine composition is substantially saponin-free. In additional embodiments, the vaccine composition is essentially free of CpG deoxyribonucleotide.

The embodiments wherein the vaccine composition is essentially free of CpG deoxyribonucleotide are preferred if the antigen in the vaccine contains a whole ssRNA virus (either a (+)ssRNA or a (-)ssRNA virus) sequences that are immunostimulatory though targeting TLR7/8. Such TLR 7/8 stimulatory sequences include polyU or GU-rich ssRNA sequences. Hell F, Hemmi H. et al., 2004. Science 303(5663):1526-9. Diebold SS., Kaisho T. et al., 2004. Science 303(5663):1529-31.

The viruses containing such sequences include, without limitations, different influenza viruses (*e.g*., bovine influenza virus, canine influenza virus, equine influenza virus, swine influenza virus and the like). In particularly preferred embodiments, the antigenic component of vaccine essentially free of CpG ODN contains influenza virus.

An antigenic component may include, in different exemplary embodiments, bovine antigens, caprine antigens, porcine antigens, poultry antigens, equine antigens, canine antigens, equine antigens and feline antigens.

Antigens can be any of a wide variety of substances capable of producing a desired immune response in a subject, including, without limitations, one or more of viruses (inactivated, attenuated, and modified live), bacteria, parasites, nucleotides (including, without limitation nucleic-acid based antigens, *e.g.*, DNA vaccines or mRNA vaccines), polynucleotides, peptides, polypeptides, recombinant proteins, synthetic peptides, protein extract, cells (including tumor cells), tissues, polysaccharides, carbohydrates, fatty acids, lipoteichioc acid, peptidoglycans, lipids, or glycolipids, individually or in any combination thereof.

Antigens used with the adjuvants of the invention also include immunogenic fragments of nucleotides, polynucleotides, peptides, polypeptides, that can be isolated from the organisms referred to herein, or chemically or biologically manufactured.

Live, modified-live, and attenuated viral strains that do not cause disease in a subject have been isolated in non-virulent form or have been attenuated using methods well known in the art, including serial passage in a suitable cell line or exposure to ultraviolet light or a chemical mutagen. Inactivated or killed viral strains are those which have been inactivated by methods known to those skilled in the art, including treatment with formalin, beta-propriolactone (BPL), peroxides, binary ethyleneimine (BEI), sterilizing radiation, heat, or other such methods.

Two or more antigens can be combined to produce a polyvalent composition that can protect a subject against a wide variety of diseases caused by the pathogens. Currently, commercial manufacturers of vaccines, as well as end users, prefer polyvalent vaccine products. While conventional adjuvants are often limited in the variety of antigens with which they can be effectively used (either monovalently or polyvalently), the adjuvants described herein can be used effectively with a wide range of antigens, both monovalently and polyvalently. Thus, the antigens described herein can be combined in a single composition comprising the adjuvants described herein.

Examples of bacteria which can be used as antigens with the adjuvant compositions described herein include, but are not limited to, Acinetobacter calcoaceticus, Acetobacter paseruianus, Actinobacillus pleuropneumoniae, Aeromonas hydrophila, Alicyclobacillus acidocaldarius, Arhaeglobus fulgidus, Bacillus pumilus, Bacillus stearothermophillus, Bacillus subtilis, Bacillus thermocatenulatus, Bordetella bronchiseptica, Burkholderia cepacia, Burkholderia glumae, Campylobacter coli, Campylobacter fetus, Campylobacter jejuni, Campylobacter hyointestinalis, Chlamydia psittaci, Chlamydia trachomatis, Chlamydophila spp., Chromobacterium viscosum, Erysipelothrix rhusiopathieae, Listeria monocytogenes, Ehrlichia canis, Escherichia coli, Haemophilus influenzae, Haemophilus somnus, Helicobacter suis, Lawsonia intracellularis, Legionella pneumophilia, Moraxellsa sp., Mycobactrium bovis, Mycoplasma hyopneumoniae, Mycoplasma mycoides subsp. mycoides LC, Clostridium perfringens, Odoribacter denticanis, Pasteurella (Mannheimia) haemolytica, Pasteurella multocida, Photorhabdus luminescens, Porphyromonas gulae, Porphyromonas gingivalis, Porphyromonas salivosa, Propionibacterium acnes, Proteus vulgaris, Pseudomonas wisconsinensis, Pseudomonas aeruginosa, Pseudomonas fluorescens C9, Pseudomonas fluorescens SIKW1, Pseudomonas fragi, Pseudomonas luteola, Pseudomonas oleovorans, Pseudomonas sp B11-1, Alcaliges eutrophus, Psychrobacter immobilis, Rickettsia prowazekii, Rickettsia rickettsii, Salmonella enterica all serovars, including for example: Salmonella enterica Typhimurium, Salmonella enterica Bongori, Salmonella enterica Dublin,, Salmonella enterica Choleraesuis, and Salmonella enterica Newport, Serratia marcescens, Spirluina platensis, Staphylococcus aureus, Staphylococcus epidermidis, Staphylococcus hyicus, Streptomyces albus, Streptomyces cinnamoneus, Streptococcus uberis, Streptococcus suis, Streptomyces exfoliates, Streptomyces scabies, Sulfolobus acidocaldarius, Syechocystis sp., Vibrio cholerae, Borrelia burgdorferi, Treponema denticola, Treponema minutum, Treponema phagedenis, Treponema refringens, Treponema vincentii, Treponema palladium, Trueperella pyogenes and Leptospira species, such as the known pathogens Leptospira canicola, Leptospira grippotyposa, Leptospira hardjo, Leptospira borgpetersenii hardjo-bovis, Leptospira borgpetersenii hardjoprajitno, Leptospira interrogans, Leptospira icterohaemorrhagiae, Leptospira pomona, and Leptospira bratislava, and combinations thereof.

Both inactivated viruses and attenuated live viruses may be used in the adjuvant compositions. Some examples of viruses which can be used as antigens include, but are not limited to, Avian herpesviruses, Bovine herpesviruses, Canine herpesviruses, Equine herpesviruses, Feline viral rhinotracheitis virus, Marek's disease virus, Ovine herpesviruses, Porcine herpesviruses, Porcine Epidemic Diarrhea virus (PEDv), Pseudorabies virus, Avian paramyxoviruses, Bovine respiratory syncytial virus, Canine distemper virus, Canine parainfluenza virus, canine adenovirus, canine parvovirus, Bovine Parainfluenza virus 3, Ovine parainfluenza 3, Rinderpest virus, Border disease virus, Bovine viral diarrhea virus (BVDV), BVDV Type I, BVDV Type II, Classical swine fever virus, Avian Leukosis virus, Bovine immunodeficiency virus, Bovine leukemia virus, Bovine tuberculosis, Equine infectious anemia virus, Feline immunodeficiency virus, Feline leukemia virus (FeLV), Newcastle Disease virus, Ovine progressive pneumonia virus, Ovine pulmonary adenocarcinoma virus, Canine coronavirus (CCV), pantropic CCV, Canine respiratory coronavirus, Bovine coronavirus. Feline Calicivirus, Feline enteric coronavirus, Feline infectious peritonitis, virus, Porcine epidemic diarrhea virus, Porcine hemagglutinating encephalomylelitis virus, Porcine parvovirus, Porcine Circovirus (PCV) Type I, PCV Type II, Porcine Reproductive and Respiratory Syndrome (PRRS) Virus, Transmissible gastroenteritis virus, Turkey coronavirus, Bovine ephemeral fever virus, Rabies, Rotovirus, Vesicular stomatitis virus, lentivirus, Avian influenza, Rhinoviruses, Equine influenza virus, Swine influenza virus, Canine influenza virus, Feline influenza virus, Human influenza virus, Eastern Equine encephalitis virus (EEE), Venezuelan equine encephalitis virus, West Nile virus, Western equine encephalitis virus, human Immunodeficiency virus, human papilloma virus, varicella zoster virus, hepatitis B virus, rhinovirus, and measles virus, and combinations thereof.

Examples of peptide antigens include Bordetella bronchiseptica p68, GnRH, IgE peptides, Fel dl, and cancer antigens, and combinations thereof. Examples of other antigens include nucleotides, carbohydrates, lipids, glycolipids, peptides, fatty acids, lipoteichoic and teichoic acid, and peptidoglycans, and combinations thereof.

Examples of parasites which can be used as antigens with the adjuvant compositions described herein include, but are not limited to, Anaplasma, Fasciola hepatica (liver fluke), Coccidia, Eimeria spp., Neospora caninum, Toxoplasma gondii, Giardia, Dirofilaria (heartworms), Ancylostoma (hookworms), Cooperia, Haemonchus contortus (Barber pole worm), Ostertagia ostertagi (stomach worm), Dictyocaulus viviparous (lung worms), Trypanosoma spp., Leishmania spp., Trichomonas spp., Cryptosporidium parvum, Babesia, Schistosoma, Taenia, Strongyloides, Ascaris, Trichinella, Sarcocystis, Hammondia, and Isopsora, and combinations thereof. Also contemplated are external parasites including, but not limited to, ticks, including Ixodes, Rhipicephalus, Dermacentor, Amblyomma, Boophilus, Hyalomma, and Haemaphysalis species, and combinations thereof.

The amount of antigen used to induce an immune response will vary considerably depending on the antigen used, the subject, and the level of response desired, and can be determined by one skilled in the art. For vaccines containing modified live viruses or attenuated viruses, a therapeutically effective amount of the antigen generally ranges from about 10² Tissue Culture Infective Dose (TCID)₅₀ to about 10¹⁰ TCID₅₀, inclusive. For many such viruses, a therapeutically effective dose is generally in the range of about 10² TCID₅₀ to about 10⁸ TCID₅₀, inclusive. In some embodiments, the ranges of therapeutically effective doses are about 10³ TCID₅₀ to about 10⁶ TCID₅₀, inclusive. In some other embodiments, the ranges of therapeutically effective doses are about 10⁴ TCID₅₀ to about 10⁵ TCID₅₀, inclusive.

For vaccines containing inactivated viruses, a therapeutically effective amount of the antigen is generally at least about 100 relative units per dose, and often in the range from about 1,000 to about 4,500 relative units per dose, inclusive. In other embodiments, the therapeutically effective amount of the antigen is in a range from about 250 to about 4,000 relative units per dose, inclusive, from about 500 to about 3,000 relative units per dose, inclusive, from about 750 to about 2,000 relative units per dose, inclusive, or from about 1,000 to about 1,500 relative units per dose, inclusive.

A therapeutically effective amount of antigen in vaccines containing inactivated viruses can also be measured in terms of Relative Potency (RP) per mL. A therapeutically effective amount is often in the range from about 0.1 to about 50 RP per mL, inclusive. In other embodiments, the therapeutically effective amount of the antigen is in a range from about 0.5 to about 30 RP per mL, inclusive, from about 1 to about 25 RP per mL, inclusive, from about 2 to about 20 RP per mL, inclusive, from about 3 to about 15 RP per mL, inclusive, or from about 5 to about 10 RP per mL, inclusive.

The number of cells for a bacterial antigen administered in a vaccine ranges from about 1x10⁶ to about 5x10¹⁰ colony forming units (CFU) per dose, inclusive. In other embodiments, the number of cells ranges from about 1x10⁷ to 5x10¹⁰ CFU/dose, inclusive, or from about 1x10⁸ to 5x10¹⁰ CFU/dose, inclusive. In still other embodiments, the number of cells ranges from about 1x10² to 5x10¹⁰ CFU/dose, inclusive, or from about 1x10⁴ to 5x10⁹ CFU/dose, inclusive, or from about 1x10⁵ to 5x10⁹ CFU/dose, inclusive, or from about 1x10⁶ to 5x10⁹ CFU/dose, inclusive, or from about 1x10⁶ to 5x10⁸ CFU/dose, inclusive, or from about 1x10⁷ to 5x10⁹ CFU/dose, inclusive.

The number of cells for a parasite antigen administered in a vaccine ranges from about 1x10² to about 1x10¹⁰ per dose, inclusive. In other embodiments, the number of cells ranges from about 1x10³ to about 1x10⁹ per dose, inclusive, or from about 1x10⁴ to about 1x10⁸ per dose, inclusive, or from about 1x10⁵ to about 1x10⁷ per dose, inclusive, or from about 1x10⁶ to about 1x10⁸ per dose, inclusive.

### Excipients

Adjuvant formulation and/or vaccine compositions may include a pharmaceutically acceptable carrier. As used herein, "a pharmaceutically-acceptable carrier" includes any and all solvents, dispersion media, coatings, stabilizing agents, diluents, preservatives, antibacterial and antifungal agents, isotonic agents, adsorption delaying agents, and the like. The carrier(s) must be "acceptable" in the sense of being compatible with the other components of the compositions and not deleterious to the subject. Typically, the carriers will be sterile and pyrogen-free, and selected based on the mode of administration to be used. It is well known by those skilled in the art that the preferred formulations for the pharmaceutically acceptable carrier which comprise the compositions are those pharmaceutical carriers approved in the applicable regulations promulgated by the United States (US) Department of Agriculture or US Food and Drug Administration, or equivalent government agency in a non-US country. Therefore, the pharmaceutically accepted carrier for commercial production of the compositions is a carrier that is already approved or will be approved by the appropriate government agency in the US or foreign country.

### Administration of the Compositions

Dose sizes of compositions typically range from about 1 mL to about 5 mL, inclusive, depending on the subject and the antigen. For example, for a canine or feline, a dose of about 1 mL is typically used, while in cattle a dose of about 2-5 mL is typically used. However, these adjuvants also can be formulated in microdoses, wherein doses of about 100 µL can be used.

The routes of administration for adjuvant compositions include parenteral, oral, oronasal, intranasal, intratracheal, subcutaneous, intramuscular, transcutaneous, intradermal, intraperitoneal, intraocular, intravenous administration and in ova. Any suitable device may be used to administer the compositions, including syringes, droppers, needleless injection devices, patches, and the like. The route and device selected for use will depend on the composition of the adjuvant, the antigen, and the subject and such are well known to the skilled artisan.

### Use of the Compositions

The adjuvant formulations described herein are easy to manufacture and stable for at least 18 months at 4 °C Formulations may be stable, for example, for about 18 months, or about 18 to about 24 months at 4 °C. In another embodiment the formulations are stable for at least about 24 months at 4 °C. Accelerated testing procedures also indicate that the formulations described herein are stable for at least two weeks at 37 °C which corresponds to about 24 months at 4 °C.

The adjuvant compositions described herein can be safely and effectively administered to a wide range of subjects. It has been surprisingly found that the adjuvant compositions described herein demonstrate safety improvements when compared with other adjuvant compositions.

The following examples are presented as illustrative embodiments, but should not be taken as limiting the scope of the invention. Many changes, variations, modifications, and other uses and applications of this invention will be apparent to those skilled in the art.

### EXAMPLES

### Examp/e 1 - Characterization of Liposomes

DCRL liposomes (containing DDA, Cholesterol, Bay®R1005, and Lecithin) were adjusted to a final concentration of 50, 50, 100 and 100 µg/mL of DDA, cholesterol, R1005 and soy lecithin, respectively.

The compounds were added to a 250 mL round bottom flask and filled to a final volume of 5 mL with anhydrous grade chloroform (Sigma-Aldrich, Poole, Dorset, UK) using 1 mL Solvent Safe™ pipettor tips (Sigma-Aldrich). Solvent was removed using a rotary evaporator (Büchi, Flawil, Switzerland) at 55 °C under vacuum (KNF Neuberger, Witney, Oxfordshire, UK) for 1 hour until a dry, white lipid film formed. Liposomes were rehydrated with either double distilled water (ddH₂O) from an in-house modified Option 3 water purifier including reverse osmosis for ultra-high purity (ELGA LabWater, Wycombe, Buckinghameshire, UK) or phosphate-buffered saline (PBS; Sigma-Aldrich) reconstituted from tablets with ddH₂O.

After rehydration, liposomes were handled with 1 mL wide orifice pipettor tips (VWR, Lutterworth, Leicestershire, UK) to reduce shearing. Then, the liposome suspension was sonicated in a bath (Sarose Scientific Instruments, Perivale, Middlesex, UK) for 1 hour and/or extruded up to 3 times through a 100 nm polycarbonate Whatman® Nucleopore Track-Etched Membrane (Sigma-Aldrich) using a mini extruder cell fitted with two 1 mL syringes. Polycarbonate membrane was flanked by 10 mm filter supports.

Sonicated DCRL liposomes (1.14 µm average diameter) were significantly larger (5.44-fold) than extruded liposomes. Sonication followed by extrusion did not significantly affect vesicle size compared to extruded samples (209.6 nm). Polydispersity of extruded liposomes was 2.10-fold lower than sonicated liposomes. A combination of sonication and extrusion significantly increased DCRL polydispersity 1.42-fold compared to extruded liposomes.

To evaluate the colloidal stability of liposomes in aqueous solution, DCRL liposomes hydrated in ddH₂O were assessed over 4 weeks. Notably, an approximate thermodynamic equivalent of 2 years at 4 °C is storage for 4 weeks at 37 °C, thus motivating assessment of product stability at both 4 and 37 °C. Both empty ["-ODN") DCRL liposomes and DCRL liposomes loaded with an exemplary immunostimulatory oligonucleotide CpG ODN ("+ODN") were assessed to determine the effect of anionic nucleic acids on cationic liposomal colloidal stability. Here, ODN was loaded by rehydrating lipid film with a solution of ddH₂O and ODN. Size and polydispersity of DCRL liposomes hydrated in ddH₂O (177 nm, 0.24) were statistically similar to those hydrated in PBS (210 nm, 0.17).

At 4°C, +/-ODN DCRL liposomes underwent insignificant 1.10- and 1.01-fold increases in diameter after 28 days, respectively. Liposomes containing ODN (+ODN liposomes) (417 nm) were significantly larger than liposomes lacking ODN (-ODN liposomes) (177 nm). Similarly, polydispersity decreased modestly 1.08- and 1.03-fold for +/-ODN liposomes after 28 days, respectively, though polydispersity at day 28 was significantly higher in +ODN liposomes (1.23-fold) compared to -ODN counterparts.

Conversely, aggregation behavior of the liposomes was observed after 28 days when stored at 37 °C. +/-ODN liposomes underwent significant 3.24- and 2.00-fold increases in diameter after 28 days, respectively, but stabilized after day 21. While +ODN liposomes were larger (1.46-fold at day 28) than -ODN liposomes owing to ODN-mediated charge compensation and loss of colloidal stability, stability trends over time were qualitatively similar. However, destabilization of +ODN liposomes occurred after day 7, whereas in -ODN liposomes, stability was maintained until after day 14. Additionally, polydispersity of +/-ODN liposomes were statistically similar after 28 days, having undergone significant 1.92- and 1.50-fold reductions, respectively, in polydispersity compared to vesicles at day 0.

These results suggest the need for development of technologies for preservation of the liposome and also suggest that more powerful sonication and/or extrusion techniques resulting in an initially smaller liposome size may compensate for initial aggregation of liposomes.

Sizes of liposomes prepared using microfluidization (Microfluidics Corp., Model 110EH) and sonication were compared. When the step of sonication was replaced with microfluidization, the formulation yielded liposomes having mean diameter of about 59 nm.

These results demonstrate that liposomes prepared using microfluidization can minimize the aggregation of the liposomes which may potentially be caused by the addition of ODN or ORN.

### Example 2 - Stability of lyophilized Liposomes

Lyophilization was performed using a Dura-Stop freeze-dryer (SP Scientific, Ipswich, Suffolk, UK). Samples were filled into 10 mL tubular type I glass freeze-drying vials (Schott, Stafford, Staffordshire, UK) prior to lyophilization. The lyophilization cycle parameters were as follows. Samples were frozen for 30 minutes at 5 °C, 30 minutes at -5 °C and 60 minutes at -40 °C, all at a ramp rate of 1.00 °C/min. Primary drying was performed for 59 minutes at -37 °C, 59 minutes at -28 °C, 59 minutes at -23 °C and 552 minutes at -21 °C, all at a ramp rate of 0.50 °C/min. Secondary drying was performed at 20 °C for 360 minutes at a ramp rate of 0.10 °C/min. All drying was performed with a chamber pressure of 57 mTorr. An alternate primary drying cycle was performed at 57 mTorr for 59 minutes at -38 °C, 59 minutes at -38 °C, 59 minutes at -37 °C and 552 minutes at -35 °C, all at a ramp rate of 0.50 °C/min. Lyophilized samples were sealed with 14 mm pharmaceutical grade butyl rubber freeze-dry stoppers (Fisher Scientific, Loughborough, Leicestershire, UK) and Parafilm M° (Sigma-Aldrich).

Lyoprotectants D-mannitol, D-(+)-glucose, D-(-)-fructose, sucrose, D-(+)-trehalose dihydrate (Sigma-Aldrich) or D(+)-mannose (Acros Organics) were solubilized in ddH₂O or PBS and added to liposome suspension to a final concentration of 2-4% w/v prior to lyophilization as indicated.

in the absence of sugars, ddH₂O- and PBS-rehydrated liposomes were ∼210 nm before lyophilization. Without lyoprotectants, lyophilization increased liposome size by 3.55- and 6.05-fold in ddH₂O-and PBS-rehydrated samples, respectively. DCRL liposomes rehydrated in ddH₂O or PBS did not undergo significant size changes after addition of 3% sucrose, 4% mannitol or a combination, demonstrating that aggregation observed post-lyophilization was not caused directly by addition of the lyoprotectant(s).

For liposomes rehydrated in PBS, liposome size was significantly increased (2.00-fold) only in samples without lyoprotectant after the freeze cycle. However, after the full lyophilization cycle, liposome size was significantly increased 6.05-, 3.03-, 5.15- and 4.13-fold for samples without lyoprotectant, with 3% sucrose, with 4% mannitol and with a combination, respectively. Similarly, polydispersity was significantly increased by 2.61-, 2.61-, 2.85- and 2.79-fold for DCRL without lyoprotectant, with 3% sucrose, with 4% mannitol and with a combination, respectively, compared to pre-lyophilized controls.

For liposomes rehydrated in ddH₂O, vesicle size after the freeze cycle was increased significantly over pre-lyophilization controls by 17.90- and 11.45-fold for liposomes without lyoprotectants and with 4% mannitol, respectively, while liposomes with 3% sucrose or a combination of 3% sucrose and 4% mannitol underwent negligible changes. After the full lyophilization cycle, liposome sizes modestly changed 3.55-, 0.88-, 3.48- and 1.47-fold for samples without lyoprotectant, with 3% sucrose, with 4% mannitol and with a combination, respectively.

These findings demonstrate that liposomes rehydrated in ddH₂O have better colloidal stability in comparison to those rehydrated in PBS as illustrated by unchanged vesicle size post-lyophilization and that 3% sucrose may be a more ideal lyoprotectant for conferring good colloidal stability.

Toward the optimization of a lyophilization scheme for ddH₂O-rehydrated DCRL liposomes, the effect of 6 known sugar lyoprotectants (individually and in combination), at 2% w/v on vesicle size and polydispersity were evaluated.

Sucrose, glucose, mannitol, trehalose, fructose and mannose were tested as lyoprotectants. Only 2% mannitol as lyoprotectant significantly increased vesicle size (39.66-fold) and polydispersity (2.95-fold). However, while lyophilization with all other sugars did not result in appreciable size change, products lyoprotected with glucose, fructose and/or mannose collapsed into impermeable, compact layers of sugar and liposomes, which were difficult to rehydrate. Consistent with literature, liposomes lyoprotected with sucrose or trehalose prevented particle size change and resulted in a cake which could be reconstituted into a liposomal dispersion with good colloidal stability.

DCRL was also lyophilized with combinations of sugars. This study revealed that only 2% sucrose, 2% trehalose and 2% sucrose/2% trehalose supported good colloidal stability post-lyophilization and achieved pharmaceutically elegant cake structure. However, the combination of 2% sucrose/2% trehalose did not appear to be advantageous in terms of liposome size stability and polydispersity over lyoprotection with 2% sucrose or 2% trehalose alone.

### Example 3 -IBR/ BVDV vaccine

Quil A adjuvant has been reported to cause a systemic immune response often accompanied by a transient fever. Such fever is believed to be associated with milk yield drop in lactating cows. The objective of this study was to evaluate the effects of saponin-free liposomes on the immune response elicited by the liposomes and potential side effects caused by the adjuvants.

Eight-month old Holstein male calves were used for this study. Potential test animals were serologically screened and those with serum neutralization (SN) titers <1:2 for BVDV-1 and BVDV-2 were eligible for enrollment in the study. In addition, animals were not persistently infected (PI) with BVDV as determined by ear punch biopsy and Immunohistochemistry. The animals were housed in environment controlled for temperature and humidity and received commercial deed and city-system water ad libitum.

Acclimation began about one week prior to study day 0. Calves received DRAXXIN® and DECTOMAX® prior to shipment.

Test animals that become moribund, Injured, or died due to conditions unrelated to the investigation were excluded from the study and the relevant data analysis. Moribund animals were euthanized.

Animals were vaccinated on days zero and 28 by subcutaneous administration of 2 ml of the vaccine as summarized in Table 1.

**Table 1**

| Grp | N | VACCCINATION | |
|---|---|---|---|
| | | Vaccine | Adjuvant (per dose) |
| T01 | 9 | Saline | None |
| T02 | 9 | Quil A Containing Adjuvant +mBVDV ½+mIBR | Quil A (250 µg), Cholesterol (250 µg), DDA (100 µg), CARBOPOL® (0.0375% v/v), BAYR1005®acetate (1,000 µg), CpG (SEQ ID NO: 8,65% homogeneity, 100 µg) |
| T03 | 9 | DCRL+mBVDV ½+mIBR | Liposomes containing Cholesterol (250 µg), DDA (250 µg), R1005 acetate (500 µg), Lecithin (500 µg) |
| T04 | 9 | DCRL-ORN (low) +mBVDV ½+mIBR | DCRL liposomes as in T03, 25 µg ORN. |
| T05 | 9 | DCRL+CpG+ORN +mBVDV ½+mIBR | DCRL liposomes as In T03, 100 µg ORN (SEQ ID NO: 11, with phosphorotioate bonds), CpG (SEQ ID NO: 8, 65% homogeneity). |
| T06 | 9 | DCRL-ORN (high) +mBVDV ½+mIBR | DCRL liposomes as in T03, 100 µg SEQ ID NO: 11, with phosphorotioate bonds |
| T07 | 6 | Quil A Containing Adjuvant +BVDV1 r-gp53 | Adjuvant as in T02 |

On day 49, animals were challenged with Noncytopathic Bovine Viral Diarrhea Virus Type 2 (Strain 24515). Each animal received approximately 4.88 Log₁₀TCID₅₀ per dose in 5 ml administered intranasally (2.5 ml per nostril) using a compressed gas atomizer.

In groups T02-T06, the antigenic component contained 4500RUs/virus pre-inactivated modified BVDV ½ and modified IBR (8.0 log₁₀TCID₅₀).

Animals were challenged on day 48 with 5 ml BVDV-2 strain 24515 (4.88 Log₁₀TCID₅₀ per dose) Intranasally (2.5 ml per nostril) using a compressed gas atomizer.

Rectal temperature least square means and ranges post-first and second vaccinations are shown in Table 2.

**Table 2. Analysis of Least Squares Mains for Rectal Temperatures Vaccination Phase**

| Grp | Time period, Day | | | | | | | | | |
|---|---|---|---|---|---|---|---|---|---|---|
| | 0 | 1 | 2 | 3 | 7 | 28 | 29 | 30 | 31 | 35 |
| T01 | 102.4^{a} | 102.0^{ab} | 101.6^{a} | 101.6^{a} | 102.3^{a} | 102.6^{ab} | 101.9^{a} | 101.8^{ab} | 101.9^{b} | 101.8^{a} |
| T02 | 102.5^{a} | 103.4^{c} | 101.6^{a} | 101.6^{a} | 102.5^{a} | 102.2^{ab} | 103.4^{c} | 102.2^{c} | 101.8^{ab} | 102.1^{b} |
| T03 | 102.4^{a} | 102.2^{ab} | 101.7^{a} | 101.5^{a} | 102.2^{a} | 102.4^{ab} | 101.9^{a} | 102.0^{bc} | 101.8^{ab} | 101.9^{ab} |
| T04 | 102.5^{a} | 102.0^{ab} | 101.8^{a} | 101.8^{a} | 102.3^{a} | 102.8^{b} | 101.9^{a} | 101.8^{ab} | 101.8^{ab} | 101.8^{a} |
| T05 | 102.2^{a} | 101.8^{a} | 101.6^{a} | 101.7^{a} | 102.0^{a} | 102.4^{ab} | 102.0^{a} | 101.8^{ab} | 101.8^{ab} | 101.8^{a} |
| T06 | 102.1^{a} | 102.0^{ab} | 101.5^{a} | 101.5^{a} | 102.2^{a} | 102.5^{ab} | 101.8^{a} | 102.6^{ab} | 101.^{b} | 101.7^{a} |
| T07 | 102.3^{a} | 102.4^{b} | 101.7^{a} | 101.5^{a} | 102.1^{a} | 102.0^{a} | 102.5^{b} | 101.5^{a} | 101.6^{a} | 101.8^{a} |

| | | | | | | | | | | |
|---|---|---|---|---|---|---|---|---|---|---|
| Values with different superscripts are significantly different (p≤0.10) | | | | | | | | | | |

The rectal temperatures for some T02 and T07 subjects showed a transient Increase on day after both first and second vaccination. The increase in rectal temperatures of the animals in group T02 was significantly higher (*P≤*0.10) than in the other groups (T01, T03, T04, T05, T06, and T07) for 1 day post first vaccination.

After the first vaccination, four out of 9 animals in Group T02 had temperatures greater than 103.5 °F (three had temperatures of over 104.0 °F). In contrast, no animals in group T05 had temperatures over 102.5 °F one day after the first vaccination (data not shown).

After second vaccination Increase in rectal temperatures of the T02 and T07 was significantly (*P*≤0.10) higher than the controls and vaccinates (T03, T04, T05, and T06), however, the elevated rectal temperatures were short lived (Table 2). Individual responses in group T02 ranged between 101.1 and 104.5 °F. Five out of nine animals In Group T02 had temperatures over 103.5 °F (and another animal had temperature 103.5 °F) after the second vaccination. Only three out of nine animals in group T02 had temperature below 103.5 °F one day after the second vaccination. In contrast, none of the DCRL formulations induced an elevation of rectal temperatures greater than 103.7 °F. Individual responses In Group T05 ranged between 101.4 and 102.6 °F (data not shown).

A febrile reaction was observed (>104.5°F) in 8 of 8 animals in the T01 group post-challenge; this met the outcome criteria for a successful challenge. Rectal temperatures of all vaccinated groups were significantly lower (*P≤*0.10) on day 53 of study compared to the controls (4 days post-challenge) and there were some differences on subsequent days (data not shown). In the control group, in response to challenge, a typical biphasic elevation of the rectal temperature was observed.

The presence of clinical BVDV disease (an animal had to have a clinical score of ≥ 2) post-challenge was determined according to the following scoring system:
0 - no clinical signs
1 -Clinical signs as a whole are not specific for acute BVD infection. Clinical signs may include nasal discharge, abnormal respiration and mild lethargy.
2 -Clinical signs as a whole are moderate in degree and specific for acute BVD infection. Clinical signs may include nasal discharge, abnormal respiration, lethargy, gauntness, ocular discharge, hypersalivation, diarrhea, dehydration, lameness and/or reluctance to move.
3 -Clinical signs as a whole are severe in degree and characteristic for acute BVD infection. Clinical signs may include nasal discharge, abnormal respiration, lethargy, gauntness, ocular discharge, hypersalivation, diarrhea, excessive bruising, dehydration, recumbency, lameness and/or reluctance to move.

There were no significant differences between the control group and the vaccinated groups.

### Leukopenia

The study outcome met the criteria for a valid study as 100% of T01 (controls) had leukopenia when using ≥ 40% drop and 75% of the controls had <4000 cells/µL. There were no significant differences between the number of animals that developed leukopenia ≥ 40% decrease In white blood cells from background post-challenge in T01 compared with the vaccinated treatment groups T02, T03, T04, T05, T06, and T07 (*P≤*0.10). However, clinical leukopenia (<4000 cells/µL) which is a more relevant definition was detected in 6 of 8 (75%), 2 of 9 (22.2%), 1 of 8 (12.5%), 3 of 9 (33.3%), 4 of 9 (44.4%), 4 of 9 (44.4%), and 0 of 6 in T01, T02, T03, T04, T05, T06, and T07, respectively (Table 3). The duration of leukopenia was significantly longer (*P≤*0.10) in the T01 group when using ≥40% drop compared to all vaccinates and significantly longer (P*≤*0.10) when using <4000 µl compared to the vaccinated groups T02, T03, T05, T06, and T07 (Table 4).

**Table 3 Summary of Leukopenia Challenge Phase**

| Grp | Leukopenia (drop 40% or more) | | | | Leukopenia (<4000 cells per microliter) | | | |
|---|---|---|---|---|---|---|---|---|
| | No | | Yes | | No | | Yes | |
| | N | % | N | % | N | % | N | % |
| T01 | 0 | 0 | 8 | 100 | 2 | 25 | 6 | 75 |
| T02 | 1 | 11.1 | 8 | 88.9 | 7 | 77.8 | 2 | 22.2 |
| T03 | 2 | 25 | 6 | 75 | 7 | 87.5 | 1 | 12.5 |
| T04 | 1 | 11.1 | 8 | 88.9 | 6 | 66.7 | 3 | 33.3 |
| T05 | 0 | 0 | 9 | 100 | 5 | 55.6 | 4 | 44.4 |
| T06 | 0 | 0 | 9 | 100 | 5 | 55.6 | 4 | 44.4 |
| T07 | 2 | 33.3 | 4 | 66.7 | 6 | 66.7 | 0 | 0 |

**Table 4. Duration of Leukopenia**

| Grp | (drop 40% or more) | | | | <4000 cells per microliter | | | |
|---|---|---|---|---|---|---|---|---|
| | LS mean days | Standard error | Lower 90% Cl | Upper 90% Cl | LS mean days | Standard error | Lower 90% Cl | Upper 90% Cl |
| T01 | 9.4^{a} | 0.818 | 8 | 10.7 | 3.6⁸ | 1.179 | 1.4 | 5.9 |
| T02 | 3.9^{bc} | 0.771 | 2.6 | 5.2 | 0.7^{bc} | 0.522 | -0.2 | 1.6 |
| T03 | 3.0^{bc} | 0.818 | 1.6 | 4.4 | 0.1^{b} | 0.095 | 0 | 0.3 |
| T04 | 3.4^{bc} | 0.771 | 2.2 | 4.7 | 0.7^{bc} | 0.522 | -0.2 | 1.6 |
| T05 | 4.4^{bc} | 0.771 | 3.3 | 5.7 | 1.7^{ac} | 0.799 | 0.2 | 3.2 |
| T06 | 4.6^{b} | 0.771 | 3.3 | 5.8 | 1.1^{c} | 0.522 | 0.2 | 2.0 |
| T07 | 2.5^{c} | 0.944 | 0.90 | 4.1 | 0^{b} | 0.11 | -0.2 | 0.2 |

| | | | | | | | | |
|---|---|---|---|---|---|---|---|---|
| Values with different superscripts are significantly different (p≤0.10) | | | | | | | | |

### Viremia

All experimental vaccines protected against viremia. In some groups there was complete protection (T02 & T05 both containing CpG) and in others partial (T03, T04, T06, and T07). The number of viremic animals in T01 was significantly higher than the number in T02, T03, T04, T05, T06 and T07 (*P*≤0.10) (Table 5). No viremia was seen in the T02 and T05 groups. However, there was a difference between number of viremic animals in groups T03, T04, and T06 which contained no ORN, low dose ORN and high dose ORN when compared to the controls.

**Table 5. Summary of Virus Isolation Challenge Phase**

| Grp | Sample Ever Positive | | | |
|---|---|---|---|---|
| | Yes | | No | |
| | Number | % | Number | % |
| T01 | 0 | 0 | 8 | 100^{a} |
| T02 | 9 | 100 | 0 | 0^{c} |
| T03 | 6 | 75 | 2 | 25^{bc} |
| T04 | 4 | 44.4 | 5 | 55.6^{b} |
| T05 | 9 | 100 | 0 | 0^{c} |
| T06 | 4 | 44.4 | 5 | 55.6^{b} |
| T07 | 1 | 16.7 | 5 | 83.3^{ab} |

| | | | | |
|---|---|---|---|---|
| Values with different superscripts are significantly different (p≤0.10) | | | | |

The duration of viremia for the T01 group was significantly greater than all the vaccinated groups (T02 through T07) (*P*≤0.1) (Table 6). Calves in T07 had received a vaccine containing a Quil A containing adjuvant + recombinant gp53 antigen of BVDV-1 virus and were also partially protected and had a significantly shorter duration of viremia compared with T01. For these calves the challenge was with a different biotype of BVDV (type 2) virus which reflects that there was partial cross-protection between the BVDV-1 gp53 antigen and the challenge strain.

**Table 6 Days with positive virus shedding**

| Grp | LS Mean days | Standard error | Lower 90% CI | Upper 90% CI |
|---|---|---|---|---|
| T01 | 8.1^{a} | 0.451 | 7.4 | 8.9 |
| T02 | 0^{d} | 0.168 | -0.3 | 0.3 |
| T03 | 0_{.}4^{cd} | 0.178 | 0.1 | 0.7 |
| T014 | 1.1^{bc} | 0.426 | 0.4 | 1.8 |
| T05 | 0^{d} | 0.168 | -0.3 | 0.3 |
| T06 | 0.7^{c} | 0.168 | 0.4 | 0.9 |
| T07 | 2.2^{b} | 0.521 | 1.3 | 3.0 |

| | | | | |
|---|---|---|---|---|
| Values with different superscripts are significantly different (p≤0.10) | | | | |

*SN Titers*

On Day 49, prior to challenge all T02 through T07 group animals had a SN titer to BVDV-1, BVDV-2 and only T02 through T06 had antibodies to IBR antigen of ≥1:8. All vaccines were considered to have adequate potency as they met the requirement of ≥1:8. The post first and second vaccination least square means of SN titers to BVDV-1 and 2 (Day 28 and 49) are shown in Table 7. All vaccinated animals had significantly higher SN titers than the control group and there were significant differences between SN titers of vaccinate groups. The liposomal vaccines (T03 though T06) induced significantly lower SN titers to BVDV-1 and 2 than the vaccine adjuvanted with a composition containing Quil A (T02). Addition of ORNS or CpG to these formulations did not enhance these responses. The gp53 vaccine adjuvanted with a composition containing Quil A also induced high SN titers by day 49 to the BVDV-1 antigen but lower to BVDV-2 antigen. The BVDV-1 SN titers of the T07 group were not significantly different from the titers of T02 but the BVDV-2 titers were. After the BVDV-2 challenge, the SN titers of all vaccinated groups were boosted whereas the T01 group calves developed primary antibody responses (day 63).

**Table 7. Least Square Means BVDV-1a and BVDV2 SN titer**

| Grp | BVD-1a | | | | BVD-2 | | | |
|---|---|---|---|---|---|---|---|---|
| | Day 0 | Day 28 | Day 29 | Day 63 | Day 0 | Day 28 | Day 29 | Day 63 |
| T01 | 1^{a} | 1^{a} | 1^{a} | 11^{a} | 1^{a} | 1^{a} | 1^{a} | 450^{a} |
| T02 | 1^{a} | 29^{d} | 1896^{c} | 29193^{d} | 1^{a} | 25^{c} | 878^{d} | 96321^{e} |
| T03 | 1^{a} | 3^{b} | 512^{b} | 11337^{bc} | 1^{a} | 3^{b} | 354^{c} | 36521^{cd} |
| T04 | 1^{a} | 4^{bc} | 621^{b} | 8513^{b} | 1^{a} | 5^{b} | 293^{c} | 22292^{C} |
| T05 | 1^{a} | 7^{c} | 492^{b} | 22295^{cd} | 1^{a} | 7^{b} | 376^{c} | 53618^{d} |
| T06 | 1^{a} | 6^{bc} | 767^{b} | 15765^{C} | 1^{a} | 6^{b} | 355^{c} | 54058^{d} |
| T07 | 1^{a} | 6^{bc} | 2814^{c} | 35734^{d} | 1^{a} | 1^{a} | 128^{b} | 10624^{b} |

| | | | | | | | | |
|---|---|---|---|---|---|---|---|---|
| Values with different superscripts are significantly different (p≤0.10) | | | | | | | | |

**Table 8 Least Square Means IBR SN titer**

| Grp | IBR | | | |
|---|---|---|---|---|
| | Day 0 | Day 28 | Day 29 | Day 63 |
| T01 | 1^{a} | 1^{a} | 1^{a} | 1^{a} |
| T02 | 1^{a} | 9^{b} | 149^{b} | 94^{b} |
| T03 | 1^{a} | 2^{c} | 21^{c} | 15^{c} |
| T04 | 1^{a} | 3^{d} | 24^{c} | 15^{c} |
| T05 | 1^{a} | 3^{cd} | 21^{c} | 14^{c} |
| T06 | 1^{a} | 3^{cd} | 19^{c} | 13^{c} |
| T07 | 1^{a} | 1^{a} | 1^{a} | 1^{a} |

| | | | | |
|---|---|---|---|---|
| Values with different superscripts are significantly different (p≤0.10) | | | | |

### Injection site reactions

Least Squares means injection site reactions (ISR) for both left and right neck are shown Tables 9 and 10. On Day 1, small reactions occurred all six vaccination groups but receded. The largest reaction sites occurred T02 and T07 on day 29, 41.8 and 16.3, respectively. There were significant differences between injection site reactions on certain days. All vaccines were safe as injection site reactions resolved rapidly.

**Table 9. Least Squares mean for injection site reactions (Left Neck)**

| Grp | Left neck injection site reaction volume on day | | | | | | |
|---|---|---|---|---|---|---|---|
| | 0 | 1 | 2 | 3 | 7 | 28 | 49 |
| T01 | -0.1^{a} | -0.1^{a} | -0.1^{a} | -0.1^{a} | -0.1^{a} | -0.1^{a} | -0.1^{a} |
| T02 | 0.1^{a} | 5.7^{b} | 4_{.}3^{bc} | 7.9^{b} | 10.4^{b} | 1.3^{a} | 0.2^{a} |
| T03 | 0.0^{a} | 3.0^{ab} | 0.9^{ab} | 0.7^{a} | 1.5^{a} | 0.0^{a} | 0.0^{a} |
| T04 | 0.1^{a} | 0.6^{a} | 2.1^{abc} | 1.7^{a} | 1.9^{a} | 0.4^{a} | 0.4' |
| T05 | -0.1^{a} | 0.0^{a} | 0.1^{a} | 0.2^{a} | 0.0^{a} | -0.1^{a} | -0.1^{a} |
| T06 | 0.1^{a} | 0.6^{a} | 8.1^{d} | 1.3^{a} | 1.0^{a} | 0.1^{a} | 0.1^{a} |
| T07 | 0.0^{a} | 6.4^{b} | 6.0^{cd} | 9.8^{b} | 2.7' | 0.3^{a} | 1.1^{a} |

| | | | | | | | |
|---|---|---|---|---|---|---|---|
| Values with different superscripts are significantly different (p≤0.10) | | | | | | | |

**Table 10 Least Squares mean for injection site reactions (Right Neck)**

| Grp | Right neck injection site reaction volume on day | | | | | |
|---|---|---|---|---|---|---|
| | 28 | 29 | 30 | 31 | 35 | 49 |
| T01 | 0.0^{a} | 0.0^{a} | 0.0^{a} | 0.0^{a} | 0.0^{a} | 0.0^{a} |
| T02 | 0.0' | 41.8^{c} | 10.5^{b} | 17.0^{c} | 17.1^{b} | 1.5^{a} |
| T03 | 0.0^{a} | 2.6^{a} | 3.0^{a} | 6.5^{ab} | 5.2^{a} | 0.0^{a} |
| T04 | 0.0^{a} | 3.8^{a} | 1.1^{a} | 1.8^{a} | 1.3^{a} | 0.4^{a} |
| T05 | 0.0^{a} | 1.2^{a} | 0.7^{a} | 0.8^{a} | 0.8^{a} | 1.3^{a} |
| T06 | 0.0^{a} | 2.8^{a} | 2.0^{a} | 5.0^{ab} | 3.2^{a} | 0.8^{a} |
| T07 | 0.0^{a} | 16.3^{b} | 5.6^{ab} | 11.4^{bc} | 5.8^{a} | 0.0^{a} |

| | | | | | | |
|---|---|---|---|---|---|---|
| Values with different superscripts are significantly different (p≤0.10) | | | | | | |

*Cell Mediated Immunity*

The BVDV IFN gamma ELISPOT assay had a high background in the T01 group before challenge and hence, is considered unreliable for demonstration of induction of CMI responses by these vaccines to this antigen (data not shown). However the assays for the IBR antigens were reflective of Induction of a CMI by the respective vaccines (see Tables 11-13, responses to killed IBR antigen, IBR gB and gD peptide recall responses of T02 through T06). In general the highest responses were in the T02 vaccine group and T05 group that had CpG in the formulation. Post-challenge responses were not enhanced as the challenge was a BVDV virus challenge. Presumably, similar CMI responses were induced by the vaccines to the BVDV antigen.

**Table 11**

| Grp | IBR gB peptides IFN-gamma **(SFC on** day) | | | |
|---|---|---|---|---|
| | 0 | 7 | 36 | 58 |
| T01 | 0.6^{a} | 157.8^{b} | 115.3^{a} | 15.6^{a} |
| T02 | 0.6^{a} | 235.6^{b} | 459.7^{cd} | 215.3^{b} |
| T03 | 4.1^{a} | 280.0^{b} | 518.8^{d} | 141.3^{ab} |
| T04 | 0.6^{a} | 162.5^{b} | 317.8^{b} | 42.8^{a} |
| T05 | 2.5^{a} | 307.2^{b} | 612.8^{d} | 103.3^{ab} |
| T06 | 1.9^{a} | 274.4^{b} | 328.9^{ba} | 70.0^{ab} |
| T07 | 13^{a} | 82.9^{a} | 97.1^{a} | 28.8^{a} |

| | | | | |
|---|---|---|---|---|
| Values with different superscripts are significantly different (p≤0.10) | | | | |

**Table 12**

| Grp | IBR gD peptides IFN-gamma (SFC on day) | | | |
|---|---|---|---|---|
| | 0 | 7 | 36 | 58 |
| T01 | 0.0^{a} | 127.8^{a} | 135.3^{a} | 11.3^{a} |
| T02 | 1.1^{a} | 209.2^{b} | 562.2^{c} | 127.2^{a} |
| T03 | 1.3^{a} | 150.0^{ab} | 401.3^{bc} | 117.8^{a} |
| T04 | 1.7^{a} | 139.7^{b} | 301.1^{b} | 76.1^{a} |
| T05 | 2.8^{a} | 244.2^{b} | 402.2^{bc} | 54.7^{a} |
| T06 | 5.0^{a} | 195.8^{b} | 338.3^{b} | 70.6^{a} |
| T07 | 3.3^{a} | 50.8^{a} | 90.4^{a} | 29.6^{a} |

| | | | | |
|---|---|---|---|---|
| Values with different superscripts are significantly different (p≤0.10) | | | | |

**Table 13**

| Grp | mIBR antigen IFN-gamma (SFC on day) | | | |
|---|---|---|---|---|
| | 0 | 7 | 36 | 58 |
| T01 | 5.6^{a} | 199.7^{a} | 210.6^{a} | 15.6^{a} |
| T02 | 0.8^{a} | 591.9^{b} | 1721.7^{c} | 215.3^{a} |
| T03 | 1.6^{a} | 411.3^{ab} | 1187.5^{b} | 141.3^{a} |
| T04 | 1.1^{a} | 489.4^{b} | 1340.8^{b} | 42.8^{a} |
| T05 | 1.4^{a} | 643.9^{b} | 1738.6^{c} | 103.3^{a} |
| T06 | 11.1^{a} | 621.7^{b} | 1129.2^{b} | 70.0^{a} |
| T07 | 2.5^{a} | 106.3^{a} | 328.3^{a} | 28.8^{a} |

| | | | | |
|---|---|---|---|---|
| Values with different superscripts are significantly different (p≤0.10) | | | | |

A vaccine effect was observed on development and duration of viremia. All experimental vaccines protected against viremia. Complete protection was observed in some groups (T02 & T05 containing CpG) while partial protection was observed in the other groups (T03, T04, T06, and T07). The rectal temperature profiles showed a transient increase one day after both first and second vaccination for treatment groups T02 and T07 compared to T01 controls. Liposomal vaccines induced lower elevations in rectal temperature compared to T02 and T07 which had Quil A containing adjuvant. This Quil A containing adjuvant typically induces a single day elevation of temperature immediately after vaccination. The latter is particularly important for dairy cows because the fever accompanying the vaccination often results in an unacceptable milk yield drop. Liposomal formulations induced robust antibody as well as T cell responses (even Liposomes without additional immunomodulators). The Liposomal vaccines induced significantly lower SN titers to BVDV-1 and 2 and IBR antigen compared with the Quil A-containing vaccines although the levels reached were considered in the protective range for these respective diseases (BVDV-1 SN titers of 1:256-512; BVDV-2 ± 256 or greater; IBR SN titer of 1:32).

In conclusion, the liposomal formulations offer a safe and new option to deliver killed antigens for diseases which require not only antibody but CMI as well for protection. The formulations were comparable to Quil-A containing vaccines in efficacy. CpG effect on efficacy was reproducible however the ORN was not effective in enhancing immunity against BVDV challenge.

### Example 4 - Swine Influenza Virus

The objective of this study was to evaluate immune response and efficacy of several SIV-vaccines (Killed Virus, pH1N1& H3N2) containing novel adjuvant formulations and immunomodulators. Efficacy was determined by both immunological parameters (humoral and cellular) and by efficacy endpoints including clinical signs, viremia, viral shedding and lung lesions.

### Animals.

Three-week old (21+/- 3 days) pigs of both sexes were used for this study. Animals had no history of exposure to PRRSV, *Mycoplasma hyopneumoniae.* Animals or their dams had no history of vaccination against or exposure to any SIV serotype. The animals arrived on site four to seven days prior to vaccination and were fed standard ration with water *ad libitum.*

The pigs were vaccinated with compositions of Table 14 on days zero (left neck) and 21 (right neck)and challenged with H3N2 virus IN/12 on day 35.

**Table 14. Experimental setup**

| **Group** | **N** | **Antigen** | Adjuvant | Dose/route |
|---|---|---|---|---|
| T01 | 10 | None | None | 2 ml IM |
| T02 | 10 | | 5% AMPHIGEN® (lecithin oil emulsion) | 2 ml IM |
| T03 | 10 | 80HA pH1N1; 120HA H3N2 | 20% AMPHIGEN® + CpG (SEQ ID NO: 8, 50 µg /dose)+CD40 agonist (Anti-CD40 monoclonal antibody, Clone 2A5C7P1G8, 250 µg/dose) | 2 ml IM |
| T04 | 10 | | 10% SP oil * | 2 ml IM |
| T05 | 10 | | 10% SP oil+ CpG (SEQ ID NO: 8, 50 µg/dose)+CD40 agonist (250 µg/dose) | 2 ml IM |
| T06 | 10 | | TXO (CpG (SEQ ID NO: 8, 50 µg/dose+DEAE Dextran (10 mg/dose) +DRAKEOL® 6VR (45% v/v), SPAN®80 (6.3% v/v) + TWEEN®80 (1.45% v/v) | 2 ml IM |
| T07 | 10 | | DCRL liposomes (Cholesterol (250 µg), DDA (250 µg), BAYR1005® acetate (500 µg), Lecithin (500 µg)) | 2 ml IM |
| T08 | 10 | | DCRL liposomes (Cholesterol (250 µg), DDA (250 µg), BAYR1005® free base(500 µg), Lecithin (500 µg)) + CpG (SEQ ID NO: 8, 50 µg/dose) + CD40 agonist (250 µg/dose) | 2 ml IM |

| | | | | |
|---|---|---|---|---|
| *4% Squalane base subsolution diluted 10x for the final product (0.4% oil) | | | | |

Blood serum was collected for hemagglutination inhibition (HAI) analysis on days 35 and 40, and whole blood collected for ELISPOT (IFNγ) analysis on days 28, 35, and 40. The pigs were sacrificed on day 40 (5 days post-challenge) and the percentage of consolidation for each lobe (left cranial, left middle, left caudal, right cranial, right middle, right caudal and accessory) was scored and recorded as a percentage between 0 and 100%.

None of the vaccinations resulted in systemic side effects or unacceptable injection side reactions (data not shown).

HAI titers are shown in Table 15.

**Table 15. HI titers (Day 35-Pre-challenge, Day 40-post challenge)**

| | CA09-H1N1 (vaccine #1) D35 | | H3N2Vac (vaccine #2) D35 | | H3N2v-IN/12 (challenge) D35 | | H3N2v-IN/12 (challenge) D40 | |
|---|---|---|---|---|---|---|---|---|
| Grp | Mean | SEM | Mean | SEM | Mean | SEM | Mean | SEM |
| T01 | 16.25^{a} | 1.83 | 17^{a} | 1.528 | <10 (nd) | na | 34^{a} | 14.47 |
| T02 | 464^{b} | 110.5 | 208^{b} | 32 | 96 | 18.09 | 755.6^{b} | 172.4 |
| T03 | 284^{b} | 67.05 | 137.8^{b} | 27.58 | 98 | 29.28 | 391.1^{b} | 113.5 |
| T04 | 552^{b} | 102.9 | 416^{b} | 64 | 220 | 54.41 | 976^{b} | 155.4 |
| T05 | 232^{b} | 55.23 | 156^{b} | 22.67 | 54 | 7.333 | 546^{b} | 163.1 |
| T06 | 432^{b} | 109.4 | 184^{b} | 31.66 | 108 | 29.24 | 848^{b} | 177 |
| T07 | 293.3^{b} | 127.2 | 435.6^{b} | 267.4 | 133.3 | 64.64 | 464^{b} | 137.9 |
| T08 | 106.7^{b} | 17.64 | 75.56^{b} | 12.37 | 28.89 | 3.514 | 124^{b} | 25.61 |

| | | | | | | | | |
|---|---|---|---|---|---|---|---|---|
| Values with different superscripts are significantly different from T01 (p≤0.10); Unpaired T test with Welch's correction (GraphPad Prism, Ver. 6.04). nd, not detectable; na, not applicable. | | | | | | | | |

Lung Lesion scores are shown In table 16.

**Table 16 Summary of lung lesion scores (Day 40)**

| Grp | Mean | SEM | Lower 90 % Cl | Upper 90% CI | N |
|---|---|---|---|---|---|
| T01 | 3.029^{a} | 0.8004 | 1.562 | 4.496 | 10 |
| T02 | 0.651^{b} | 0.4224 | -0.1232 | 1.425 | 10 |
| T03 | 0.815^{b} | 0.7331 | -0.5289 | 2.159 | 10 |
| T04 | 0.733^{b} | 0.5106 | -0.203 | 1.669 | 10 |
| T05 | 1.36^{a} | 0.7563 | -0.02643 | 2.746 | 10 |
| T06 | 0.237^{b} | 0.1926 | -0.1161 | 0.5901 | 10 |
| T07 | 0.668^{b} | 0.3254 | 0.07155 | 1.264 | 10 |
| T08 | 4.374^{a} | 1.11 | 2.339 | 6.409 | 10 |
| NTX | 0.453^{b} | 0.4161 | -0.3098 | 1.216 | 10 |

| | | | | | |
|---|---|---|---|---|---|
| ^{a}Values with different superscripts are significantly different from T01 (p≤0.10); Unpaired T test with Welch's correction (GraphPad Prism, Ver. 6.04). NTX is a group of unvaccinated, unchallenged pigs. | | | | | |

Group T04 had the highest HAI titer to one of the vaccine strains (H1N1), while T07 had the highest titer to the other strain (H3H2), even though, the titers in groups T02-T08 were significantly higher than in T01 control group.

All vaccines (except T08) tended to reduce LLS at 5 days post-challenge. Group T06 (TXO) tended to have the lowest Lung Lesion Scores.

Influenza NP protein peptide-specific IFN-gamma secreting cells and whole influenza virus-specific IFN-gamma secreting cells (both determined by ELISPOT) are provided in Tables 19 and 20, respectively. The peptides in the pools are 16-mers with a 12-amino acid overlap. The four pools contain sequence of NP peptide from N-terminus (Pool 1) to C-terminus (Pool 4). The sequences of the peptides are provided in tables 17 and 18.

**Table 17. NP peptide pools used for IFN-γ ELISPOT (pools 1 and 2)**

| NP peptide pool #1 | | NP peptide pool #2 | |
|---|---|---|---|
| SEQ ID NO: | Sequence | SEQ ID NO: I | Sequence |
| 15 | MATQGTKRSYEQMETG | 45 | RQANNGEDATAGLTHI |
| 16 | GTKRSYEQMETGGERQ | 46 | NGEDATAGLTHIMIWH |
| 17 | SYEQMETGGERQDATE | 47 | ATAGLTHIMIWHSNLN |
| 18 | METGGERQDATEIKAS | 48 | LTHIMIWHSNLNDATY |
| 19 | GERQDATEIKASVGRM | 49 | MIWHSNLNDATYQRTR |
| 20 | DATEIKASVGRMVGGI | 50 | SNLNDATYQRTRALVR |
| 21 | IKASVGRMVGGIGRFY | 51 | DATVQRTRALVRT6MD |
| 22 | VGRMVGGIGRFYIQMC | 52 | QRTRALVRTGMDPRMC |
| 23 | VGGIGRFYIQMCTELK | 53 | ALVRTGMDPRMCSLMQ |
| 24 | GRFYIQMCTELKLSDY | 54 | TGMDPRMCSLMQGSTL |
| 25 | IQMCTELKLSDYEGRL | 55 | PRMCSLMQGSTLPRRS |
| 26 | TELKLSDYEGRLIQNS | 56 | SLMQGSTLPRRSGAAG |
| 27 | LSDVEGRLIQNSITIE | 57 | GSTLPRRSGAAGAAVK |
| 28 | EGRLIQNSITIERMVL | 58 | PRRSGAAGAAVKGVGT |
| 29 | IQNSITIERMVLSAFD | 59 | GAAGAAVKGVGTIAME |
| 30 | ITIERMVLSAFDERRN | 60 | AAVKGVGTIAMELIRM |
| 31 | RMVLSAFDERRNKYLE | 61 | GVGTIAMELIRMIKRG |
| 32 | SAFDERRNKYLEEHPS | 62 | IAMELIRMIKRGINDR |
| 33 | ERRNKYLEEHPSAGKD | 63 | LIRMIKRGINDRNFWR |
| 34 | KYLEEHPSAGKDPKKT | 64 | IKRGINDRNFWRGENG |
| 35 | EHPSAGKDPKKTGGPI | 65 | INDRNFWRGENGRRTR |
| 36 | AGKDPKKTGGPIYRRV | 66 | NFWRGENGRRTRAAYE |
| 37 | PKKTGGPIYRRVDGKW | 67 | GENGRRTRAAYERMCN |
| 38 | GGPIYRRVDGKWMREL | 68 | RRTRAAYERMCNILKG |
| 39 | YRRVDGKWMRELILYD | 69 | AAYERMCNILKGKFQT |
| 40 | DGKWMRELILYDKEEI | 70 | RMCNILKGKFQTAAQR |
| 41 | MRELILYDKEEIRRVW | 71 | ILKGKFQTAAQRAMMD |
| 42 | ILYDKEEIRRVWRQAN | 72 | KFQTAAQRAMMDQVRE |
| 43 | KEEIRRVWRQANNGED | 73 | AAQRAMMDQVRESRNP |
| 44 | RRVWRQANNGEDATAG | 74 | AMMDQVRESRNPGNAE |

**Table 18. NP peptide pools used for IFN-γ ELISPOT (pools 3 and 4)**

| NP peptide pool #3 | | NP peptide pool #4 | |
|---|---|---|---|
| SEQ ID NO: | Sequence | SEQ ID NO: | Sequence |
| 75 | QVRESRNPGNAEIEDL | 105 | RGVQIASNENVEAMDS |
| 76 | SRNPGNAEIEDLIFLA | 106 | IASNENVEAMDSNTLE |
| 77 | GNAEIEDLIFLARSAL | 107 | ENVEAMDSNTLELRSR |
| 78 | IEDLIFLARSALVLRG | 108 | AMDSNTLELRSRYWAI |
| 79 | IFLARSALVLRGSVAH | 109 | NTLELRSRYWAIRTRS |
| 80 | RSALVLRGSVAHKSCL | 110 | LRSRYWAIRTRSGGNT |
| 81 | VLRGSVAHKSCLPACV | 111 | YWAIRTRSGGNTNQQR |
| 82 | SVAHKSCLPACVYGLA | 112 | RTRSGGNTNQQRASAG |
| 83 | KSCLPACVYGLAVASG | 113 | GGNTNQQRASAGQISV |
| 84 | PACVYGLAVASGHDFE | 114 | NQQRASAGQISVQPTF |
| 85 | YGLAVASGHDFEREGY | 115 | ASAGQISVQPTFSVQR |
| 86 | VASGHDFEREGYSLVG | 116 | QISVQPTFSVQRNLPF |
| 87 | HDFEREGYSLVGIDPF | 117 | QPTFSVQRNLPFERAT |
| 88 | REGYSLVGIDPFKLLQ | 118 | SVQRNLPFERATIMAA |
| 89 | SLVGIDPFKLLQNSQV | 119 | NLPFERATIMAAFSGN |
| 90 | IDPFKLLQNSQVFSLI | 120 | ERATIMAAFSGNNEGR |
| 91 | KLLQNSQVFSURPNE | 121 | IMAAFSGNNEGRTSDM |
| 92 | NSQVFSLIRPNENPAH | 122 | FSGNNEGRTSDMRTEV |
| 93 | FSLIRPNENPAHKSQL | 123 | NEGRTSDMRTEVIRMM |
| 94 | RPNENPAHKSQLVWMA | 124 | TSDMRTEVIRMMESAK |
| 95 | NPAHKSQLVWMACHSA | 125 | RTEVIRMMESAKPEDL |
| 96 | KSQLVWMACHSAAFED | 126 | IRMMESAKPEDLSFQG |
| 97 | VWMACHSAAFEDLRVS | 127 | ESAKPEDLSFQGRGVF |
| 98 | CHSAAFEDLRVSSFIR | 128 | PEDLSFQGRGVFELSD |
| 99 | AFEDLRVSSFIRGKKV | 129 | SFQGRGVFELSDEKAT |
| 100 | LRVSSFIRGKKVIPRG | 130 | RGVFELSDEKATSPIV |
| 101 | SFIRGKKVIPRGKLST | 131 | ELSDEKATSPIVPSFD |
| 102 | GKKVIPRGKLSTRGVQ | 132 | EKATSPIVPSFDMSNE |
| 103 | IPRGKLSTRGVQIASN | 133 | SPIVPSFDMSNEGSYF |
| 104 | KLSTRGVQIASNENVE | 134 | PSFDMSNEGSYFFGDN |
| | | 135 | MSNEGSYFFGDNAEEY |
| | | 136 | GSYFFGDNAEEYDS |

**Table 19 IFN-γ EUSPOT NP Peptide pools (Day 40)**

| | NP peptide pool #1 | | NP peptide pool #2 | | NP pool #3 | | NP peptide pool #4 | |
|---|---|---|---|---|---|---|---|---|
| Grp | Mean | Standard error | Mean | Standard error | Mean | Standard error | Mean | Standard error |
| T01 | 47^{a} | 9.634 | 40^{a} | 10.68 | 157^{a} | 68.03 | 65.2^{a} | 19.59 |
| T02 | 66^{a} | 16.79 | 28.4^{a} | 6.598 | 68.4^{a} | 20.38 | 40.8^{a} | 10.19 |
| T03 | 83^{a} | 34.75 | 56.8^{a} | 17.58 | 101.6^{a} | 21.91 | 120^{a} | 53.86 |
| T04 | 52.6^{a} | 24.63 | 41.2^{a} | 14.61 | 63^{a} | 16.98 | 75.8^{a} | 28.02 |
| T05 | 110.8^{b} | 29.5 | 93.6^{a} | 33.8 | 179.6^{a} | 40.35 | 74^{a} | 19.43 |
| T06 | 100.6^{a} | 49.25 | 118.4^{a} | 43.82 | 132.4^{a} | 57.12 | 53.8^{a} | 17.96 |
| T07 | 388.4^{b} | 139.1 | 140.2^{b} | 42.29 | 275.8^{a} | 103.5 | 76.2' | 19.15 |
| T08 | 61.8^{a} | 15.02 | 70.6^{a} | 15.22 | 177.4^{a} | 34.7 | 61.8^{a} | 13.76 |

| | | | | | | | | |
|---|---|---|---|---|---|---|---|---|
| ^{a}Values with different superscripts are significantly different from T01 (p≤0.10); Unpaired T test with Welch's correction (GraphPad Prism, Ver. 6.04). | | | | | | | | |

NP protein is generally highly conserved among SIV strains, and therefore, an efficient CMI response likely translates into better cross-protection potential.

**Table 20. IFN-γ ELISPOT virus recall antigen (Day 40)**

| | H3N2v-IN/12 (challenge) | | CA09-H1N1 (vaccine #1) | | H3N2Vac (vaccine #2) | |
|---|---|---|---|---|---|---|
| Grp | Mean | Standard error | Mean | Standard error | Mean | Standard error |
| T01 | 91.8^{a} | 25.4 | 30^{a} | 10.56 | 23^{a} | 5.41 |
| T02 | 216.6^{b} | 36.69 | 147^{b} | 16.6 | 312^{b} | 75.42 |
| T03 | 387^{b} | 72.43 | 231^{b} | 40.74 | 497.4^{b} | 84.63 |
| T04 | 390.8^{b} | 65.18 | 225.6^{b} | 41.22 | 513^{b} | 108.3 |
| T05 | 524.4^{b} | 80.27 | 255^{b} | 52.56 | 598.2^{b} | 87.71 |
| T06 | 419.6^{b} | 108 | 584.2^{b} | 149.6 | 491.2^{b} | 144.9 |
| T07 | 788^{b} | 164.7 | 552.8^{b} | 115 | 1041^{b} | 233.8 |
| T08 | 362^{b} | 93.1 | 151.6^{b} | 44.56 | 486.8^{b} | 130.2 |

| | | | | | | |
|---|---|---|---|---|---|---|
| ^{a}Values with different superscripts are significantly different from T01 (p≤0.10); Unpaired T test with Welch's correction (GraphPad Prism, Ver. 6.04). | | | | | | |

Group T07 (DCRL liposomes) provided the highest IFN gamma responses to three of four NP protein peptide pools and two of the three whole viruses tested. The only group with observed post-vaccination (pre-challenge) IFN-γ response was T06 (TXO) (data not shown).

Adding CD40 agonist and CpG uniformly tended to decrease titers, as well as to increase LLS, especially in the context of the liposomal formulation.

Taken as a whole, these results indicate that the vaccine adjuvanted with DCRL liposomes is as effective as formulation T02 (experimental formulation similar to a commercial vaccine) in reducing lung lesion scores. At the same time, DCRL liposomes are much more effective in activating CMI response than the other vaccines tested, thus possibly providing a broader cross-protection potential and improved duration of immunity than the commercial product.

### Example 5 Development of Immunity to Eimeria maxima following profilin vaccination

The objective of the study was to evaluate the effects of various adjuvants (Zoetis proprietary) on development of immunity to *Eimeria maxima* following profilin vaccination.

Newly hatched chicks were purchased from Longenecker's hatchery, Elizabethtown, PA. Chicks were provided with feed and water ad libitum. Birds were kept In brooder pens in *Eimeria*-free facility and transferred into large hanging cages in a separate location where they were infected and kept until the end of experimental period.

Purified profilin (50µg/dose) was mixed with different adjuvants as provided in Table 21.

**Table 21**

| **Group** | **Vaccine composition** |
|---|---|
| T01 | PBS |
| T02 | PBS |
| T03 | Profilin only (50 µg) |
| T04 | 50 µg profilin +50 µg Cholesterol + CpG (SEQ ID NO: 8; 65% purity, 5 µg/ds) |
| T05 | 50 µg profilin + Quil-A (50 µg/dose), Cholesterol (50 µg/dose), CpG (5 µ/dose) |
| T06 | 50 µg profilin + Quil-A (50 µg/dose), Cholesterol (50 µg/dose), DDA (50 µg/dose), Carbopol (0.05%), BAYR1005® free base (100 µg/dose) |
| T07 | 50 µg profilin + Quil-A (50 µg/dose), Cholesterol (50 µg/dose), DDA (50 µg/dose), Carbopol (0.05%), BAYR1005® (100 µg/dose), CpG (SEQ ID NO: 8; 65% purity, 5 µg/ds) |
| T08 | 50 µg profilin + DCRL (Lyo) (DDA (12.5 µg/ds), Cholesterol (12.5 µg/ds), BAYR1005® (25 µg/ds), Lecithin (25 µg/ds)) |
| T09 | 50 µg profilin + DCRL + CpG (SEQ ID NO: 8; 65% purity, 5 µg/ds) (Lyo) (DDA (12.5 µg/ds), Cholesterol (12.5 µg/ds), BAYR1005® (25 µg/ds), Lecithin (25 µg/ds)) |
| T10 | 50 µg profilin + CpG (SEQ ID NO: 8; 65% purity, 25 µg/ds), DEAE-Dextran (500 µg/ds), 45% v/v Drakeol 6VR, Span-80 (6.3% v/v), Tween-80 (1.45% v/v) |

Birds were immunized with two subcutaneous injections (0.5 mL per dose) of profilin plus adjuvant at day 1 and day 7 of age subsequently. Seven days following second Immunization (14 days of age), birds (Except in group T01) were challenged with 1 x 10⁴ sporulated oocysts of *Eimeria maxima.*

Body weight gain was determined on d 6 and d 15 following challenge. Oocysts shed in the feces from day 6-9 following challenge was also determined. Serum antibodies against profilin were evaluated by ELISA.

Control groups: PBS injections and no challenge (non-vaccinated unchallenged control), PBS injections and challenge on d 14 (non-vaccinated challenged control), profilin/no adjuvant and challenge on d 14 (Antigen control).

To assess lesion scores, six birds/group were killed 6 d post challenge. Approximately 20cm intestinal segments extending 10cm anterior and posterior to diverticulum were obtained and cut longitudinally. Intestinal contents were removed gently. A score ranging from 0-4 was given depending on the severity of lesions.

To assess fecal oocyst production, feces from each group (8 birds/group; 4 cages with 2 birds/cage) were collected separately from 6 to 9 days post challenge. Starting from 6 days post challenge, collection cages were set up and animal caretakers were instructed not to clean the feces. Fecal droppings were collected from each oocyst collecting cage that holds 2 birds per cage. Collecting pans were placed under each cage for 3 days starting from 6 days pi, and fecal material was collected into large plastic jars. Fecal droppings in each jar were ground in a blender with water, and two 35 ml random samples were taken from each sample. In order to count coccidia oocysts, various dilutions were made initially to determine the optimum dilutions for enumeration of oocysts for each sample. Oocysts were counted microscopically. The total number of oocysts shed per chicken was calculated using the formula: total oocysts/bird = (oocyst count x dilution factor x fecal sample volume/counting chamber volume)/number of birds per cage.

Blood samples (4 birds / group) were collected on day 9 post *Eimeria* challenge for antibody response measurements. Blood samples were allowed to clot at 4°C for 4 hr, and the sera separated. Serum samples were tested for antibodies against *Eimeria* using ELISA. Briefly, microtiter plates were coated overnight with 200ng/weM of the recombinant coccidial antigen, washed with PBS-0.05% Tween, and blocked with PBS-1% BSA. Serum dilutions were added, incubated with continuous gentle shaking, washed, and bound Ab detected with peroxidase-conjugated rabbit anti-chicken IgG (Sigma) and peroxidase-specific substrate. Optical density (OD) was measured at 450nm with a microplate reader (Bio-Rad, Richmond, CA).

All values are expressed as mean ± SEM. Differences among means were considered significant at p < 0.05.

The negative control and challenge control were significantly different at 6 days but not 15 days post-infection. Day 6 post-challenge weight loss in challenge controls as compared to negative controls was about 15%. No treatment differed from no adjuvant or challenge controls but treatments T05, T06, and T08 did not differ from the negative control either.

Just under 60% of the birds had lesion scores of 2 or higher. No formulations were significantly different from the challenge control. Group T09 was the poorest performer. T04 was the best performer.

Generally speaking a decrease of < 1 log in output is not considered biologically relevant, but can still be indicative of active immunity. Only formulations that were significantly less than the challenge control were T05 and T09.

Antibody response is not generally considered relevant to immunity to coccidiosis so may not correlate to other criteria. In the experiments above, group T07 elicited antibody response which was higher than the responses elicited either by negative (unvaccinated, uninfected birds), the positive control (non-vaccinated, infected birds), or the birds vaccinated with the antigen only. Groups T05, T06, and T08-T10 elicited antibody responses higher than the negative control (unvaccinated, uninfected birds).

The results are summarized in Table 22. Weight gain, serum antibody levels (expressed as a % of uninfected control) and lesion scores, oocyst production (expressed as a % of infected control) following immunization of birds with two subcutaneous injections of profilin plus various adjuvants at day 1 and day 7 of age and subsequent challenge with 1 X 10⁴ oocysts of *Eimeria maxima* at 14 days of age.

**Table 22**

| Grp | Weight gain, % of control | | Oocyst production, % of infected control, day 6 | Lesion score, % of Serum AB titer infected control, day 6 (OD450), % of control | |
|---|---|---|---|---|---|
| | Day 0-6 | Day 0-15 | | | |
| T01 | 100 | 100 | 0 | 0 | 100 |
| T02 | 85 | 96 | 100 | 100 | 189 |
| T03 | 86 | 96 | 83 | 63 | 193 |
| T04 | 93 | 95 | 117 | 63 | 194 |
| T05 | 88 | 87 | 63 | 53 | 231 |
| T06 | 83 | 90 | 74 | 74 | 268 |
| T07 | 92 | 96 | 83 | 42 | 310 |
| T08 | 81 | 88 | 108 | 74 | 203 |
| T09 | 86 | 95 | 56 | 95 | 205 |
| T10 | 86 | 87 | 113 | 63 269 | |

### Example 6 - BVDV/IBR vaccine adjuvanted with DCRL + CpG

In this example, adjuvanting potential of DCRL liposomes with CpG (without ORN) was accessed. In addition, different methods of loading the liposomes were compared.

The experimental setup was as follows (Table 21):

| **Trmt Group** | **N** | **Treatment** | **Adjuvant (per Dose)** |
|---|---|---|---|
| T01 | 9 | Saline Negative Control | |
| T02 | 9 | Quil A containing adjuvant (gp53 from BVDV 1 and BVDV 2) | Quil-A (250 µg), Cholesterol (250 µg), DDA (100 µg), CARBOPOL® (0.0375%v/v), BAYR1005®acetate (1,000 µg), SEQ ID NO: 8 (100 µg, 65% homogeneous) + 10µg each of recombinant BVDV 1 and 2 gp 53 |
| T03 | 9 | DCRL + T (sucrose lyoph. without antigen) + mBVDV 1, mBVDV 2 & mIBR | Cholesterol (250 µg), DDA (250µg), BAYR1005® free base (500µg), Lecithin (500µg), SEQ ID NO: 8 (100 µg, 65% homogeneous) |
| T04 | 9 | DCRL + T (not lyoph) mBVDV 1, mBVDV 2 & mIBR | As in T03 |
| T05 | 9 | DCRL + T (active load with antigen then sucrose lyoph) mBVDV 1, mBVDV 2 & mlBR | As in T03 |
| T06 | 9 | DCRL-ORN (sucrose lyoph) mBVDV 1, mBVDV 2 & mlBR | Cholesterol (250µg), DDA (250µg), BAYR1005® free base (500µg), Lecithin (500µg), SEQ ID NO: 11, with phosphorotioate bonds (100 µg) |

Vaccines in groups T03-T06 also contained mBVD1 (4,500 RU/ds) + BVDV1 (4500RUs), BVDV 2 (4500RUs), mIBR 10⁸log₁₀ TCID₅₀ (pre-inactivation dose).

Healthy Holstein calves (6-7 months old, seronegative for BVDV1, BVDV2 and IBR) were enrolled In this study (n=9/treatment group). Calves were administered 2 mL of the assigned vaccine subcutaneously on Days 0 and 28. A virulent BVDV2 challenge (4 mL (5.14 Log₁₀TCID₅₀ per dose) intranasally) was administered on Day 49. Clinical observations were performed and injection sites and rectal temperatures were measured around each vaccination. Blood samples were collected for white blood cell counts, viremia and serology through Day 63.

All calves in T02, T03, T04 and T06 achieved 1:8 titers against BVDV-la and BVDV2 before challenge, compared to 22.1 and 11.1% of the calves in T05. It is believed that due to active loading used in T5, the majority of the antigen was removed from the vaccine preparation. As such, the animals in group T05 received less antigen than the animals in groups T03, T04, and T06. Therefore, the interpretation of the findings from group T05 should be interpreted cautiously.

That being said, the BVDV-la and BVDV2 titers for T05 were significantly lower than the other vaccinated groups in Days 28, 49 and 63. No calves in T02 or T05 achieved 1:8 titers against BHV before challenge, and these groups also had significantly lower titers compared to the other vaccinated groups on Days 28, 49 and 63. The Least Square Mean for gp53-1 SFC was significantly higher for T02 compared to all other treatment groups on Days 0, 35 and 56, and the Lease Square Means for gp53-2 SFC was significantly higher for T02 on Days 35 and 56.

Viremia was observed in all calves in T01 and T05. No calves in T02 developed viremia following challenge. In groups T03, T04 and T06, 22.2, 33.3 and 33.3% of the calves developed viremia; these groups were not significantly different from T02. The duration of viremia was significantly longer in T01 and T05 compared to all other treatment group. When leukopenia was determined by a 40% reduction in WBC from baseline, 22.2% of the calves in T02 were leukopenic after challenge, while all calves in all groups were leukopenic. Calves In T01 and T05 had significantly longer duration of leukopenia compared to all other groups (8.6 days, zero days, 0.2 days, 0.7 days, 6.7 days, and 0.8 days for groups T01-T06, respectively). Only mild signs of clinical disease were observed following challenge In this study. In the T01 control group, 22.2% of the calves had clinical scores of ≥2 after challenge, while 11.1% of the calves in T05 had clinical scores of ≥2 after challenge. No calves in any other treatment group had clinical scores of ≥2 after challenge.

Transient fevers were observed in T02 on Days 1 and 29. The temperatures of all other calves were normal throughout the study.

**Table 22. LSMeans for Vaccination Phase Rectal Temperature, °F**

| Trt. | Day 00 | Day 01 | Day 02 | Day 03 | Day 07 | Day 28 | Day 29 | Day 30 | Day 31 | Day 35 |
|---|---|---|---|---|---|---|---|---|---|---|
| T01 | 102.1^{ab} | 101.9^{a} | 101.9^{a} | 101.9^{b} | 102.0^{a} | 101.9^{ab} | 102.1 | 202.0^{a} | 102.0^{a} | 102.3 |
| T02 | 101.9^{a} | 103.1^{b} | 101.7^{a} | 101.9^{a} | 102.0^{a} | 101.9^{ab} | 103.2^{b} | 102.3^{a} | 102.1^{ab} | 102.2^{b} |
| T03 | 101.9^{a} | 102.1^{a} | 101.7^{a} | 102.0^{a} | 102.3^{ab} | 101.7^{a} | 102.1^{a} | 102.1^{a} | 102.1^{ab} | 102.1^{a} |
| T04 | 101.9^{a} | 101.9^{a} | 101.7^{a} | 101.9^{a} | 102.3^{ab} | 102.2^{b} | 102.4^{a} | 102.3^{a} | 102.3^{b} | 102.4^{a} |
| T05 | 192.1^{ab} | 102.0^{a} | 101.9^{a} | 102.3^{a} | 102.5^{b} | 102.0^{ab} | 102.2^{a} | 102.0^{a} | 102.2^{ab} | 102.1^{a} |
| T06 | 102.3^{b} | 102.1^{a} | 101.7^{a} | 102.0^{a} | 102.1^{ab} | 101.8^{a} | 102.3^{a} | 102.0^{a} | 102.1^{ab} | 102.2^{a} |

| | | | | | | | | | | |
|---|---|---|---|---|---|---|---|---|---|---|
| Values with different superscripts are significantly different (p≤0.10) | | | | | | | | | | |

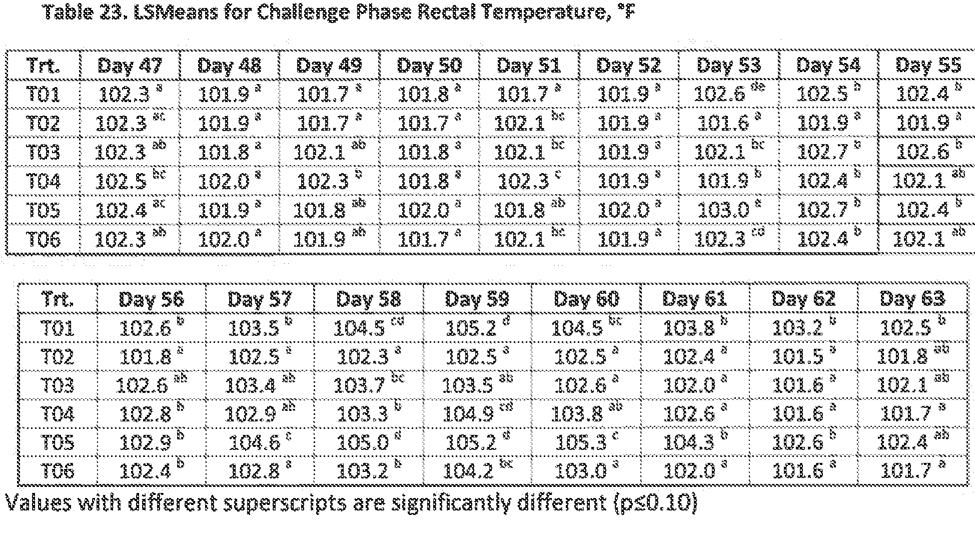

Analysis of Individual animals revealed that In group T02 (adjuvanted with a Quil A containing adjuvant), four animals had temperature over 103.5F after the first Injection and two animals had temperature over 103.5F after the second injection. In addition, one animal had temperature 103.2 F on day 1 (a day after the first vaccination) and five had temperatures in range of 103.0-103.4 F on day 29 (a day after the second vaccination). In contrast, none of the animals vaccinated with the adjuvants lacking Quil A had temperatures over 103.5 F either after the first or after the second vaccination. Two had temperatures in the range of 103.0-103.4F.

No injection site reactions >200 cm³ were observed in this study. Group T02 (adjuvanted with a Quil A - containing adjuvant) had significantly larger injection site reactions on Day 2, 7 and 29 compared to all other treatment groups. Measureable Injection site reactions were still present on Day 49 in T02 (second injection) and T06 (first Injection).

**Table 1. LSMeans for Injection Site Reactions following First Vaccination (cm³)**

| **Treatment** | **Day 00** | **Day 01** | **Day 02** | **Day 03** | **Day 07** | **Day 28** | **Day 49** |
|---|---|---|---|---|---|---|---|
| T01 | 0.0^{a} | 0.0^{a} | 0.0^{a} | 0.0^{a} | 00^{a} | 0.0^{a} | 0.0^{a} |
| T02 | 0.0^{a} | 2.0^{b} | 5.4^{c} | 5.7^{b} | 7.2^{c} | 0.2^{a} | 00^{a} |
| T03 | 0.0^{a} | 0.3^{a} | 1.8^{ab} | 1.2^{a} | 1.0^{ab} | 0.0^{a} | 0.0^{a} |
| T04 | 0.0^{a} | 0.4^{a} | 2.1^{b} | 0.0^{a} | 2.0^{ab} | 0.0^{a} | 0.0^{a} |
| T05 | 0.0^{a} | 0.0^{a} | 0.0^{a} | 0.0^{a} | 0.0^{a} | 0.0^{a} | 0.0^{a} |
| T06 | 0.0^{a} | 1.2^{a} | 3.3^{b} | 4.2^{b} | 2.1^{b} | 0.0^{a} | 0.6^{a} |

| | | | | | | | |
|---|---|---|---|---|---|---|---|
| Values with different superscripts are significantly different (p≤0.10) | | | | | | | |

**Table 25. LSMeans for Injection Site Reactions following Second Vaccination (cm³)**

| **Treatment** | **Day 28** | **Day 29** | **Day 30** | **Day 31** | **Day 35** | **Day 49** |
|---|---|---|---|---|---|---|
| T01 | 0.0^{a} | 0.0^{a} | 0.0^{a} | 0.3^{a} | 0.0^{a} | 0.0^{a} |
| T02 | 0.0^{a} | 35.8^{b} | 5.8^{b} | 5.4^{a} | 8.3^{bc} | 0.2^{a} |
| T03 | 0.0^{a} | 2.4^{a} | 0.4^{ab} | 3.6^{a} | 9.8^{c} | 0.0^{a} |
| T04 | 0.0^{a} | 0.9^{a} | 1.2^{ab} | 5.1^{a} | 1.9^{a} | 0.0^{a} |
| T05 | 0.0^{a} | 0.0^{a} | 0.0^{a} | 0.0^{a} | 0.0^{a} | 0.0^{a} |
| T06 | 0.0^{a} | 1.2^{a} | 1.2^{ab} | 3.0^{a} | 3.9^{ab} | 0.0^{a} |

| | | | | | | |
|---|---|---|---|---|---|---|
| Values with different superscripts are significantly different (p≤0.10) | | | | | | |

In sum, vaccine candidates T02, T03, T04 and T06 met the outcome criteria for providing BVDV2 titers >1:8 In all calves. Vaccine candidate T02 also provided 100% protection against viremia and complete protection against leukopenia (as measured by 40% reduction in WBC). Calves in this group experienced transient fever and injection site reactions.

## Claims

1. An essentially saponin-free liposome comprising an external lipid bilayer membrane and an internal compartment, the external membrane comprising:
a) a quaternary ammonium compound composed of four alkyl chains, two of which are C10-C20 alkyls and the remaining two are C1-C4 alkyls;
b) a sterol selected from the group consisting of β-sitosterol, stigmasterol, ergosterol, ergocalciferol, and cholesterol;
c) a phospholipid; and
d) a glycolipid of formula I: 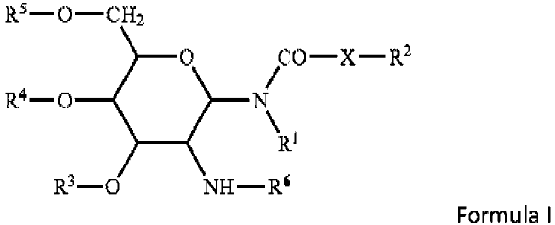
wherein, R¹ is hydrogen, or a saturated alkyl radical having up to 20 carbon atoms; X is -CH₂-, -O- or -NH-; R² is hydrogen, or a saturated or unsaturated alkyl radical having up to 20 carbon atoms; R³, R⁴, and R⁵ are independently hydrogen, - SO₄²⁻, -PO₄ ²⁻, -COC₁₋₁₀ alkyl; R⁶ is L-alanyl, L-alpha-aminobutyl, L-arginyl, L-asparginyl, L-aspartyl, L-cysteinyl, L-glutamyl, L-glycyl, L-histidyl, L-hydroxyprolyl, L-isoleucyl, L-leucyl, L-lysyl, L-methionyl, L-ornithinyl, L-phenyalany, L-prolyl, L-seryl, L-threonyl, L-tyrosyl, L-tryptophanyl, and L-valyl or their D-isomers,

2. The liposome of claim 1, wherein said liposome is saponin-free.

3. The liposome of claim 1 or 2, wherein the quaternary ammonium compound is DDA (dimethyldioctadecylammonium), the sterol is cholesterol, and the glycolipid is N-(2-Deoxy-2-L-leucylamino-β-D-glucopyranosyl)-N-octadecyldodecanoylamide or a salt thereof.

4. The liposome of any one of claims 1-3, further comprising, in the internal compartment, an immunostimulatory oligonucleotide selected from the group consisting of an immunostimulatory ribonucleotide, a CpG oligodeoxyribonucleotide, and a combination thereof.

5. The liposome of claim 4, wherein said immunostimulatory oligonucleotide comprises any one of SEQ ID NOs 1-14,

6. An adjuvant formulation comprising the liposome according to any one of claims 1-5.

7. The adjuvant composition of claim 6, wherein said adjuvant formulation is essentially saponin-free.

8. A vaccine composition comprising an effective amount of an antigenic component and the adjuvant formulation of claim 6 or claim 7.

9. The vaccine composition of claim 8, wherein said vaccine composition is essentially saponin-free.

10. The vaccine composition of claim 8 or 9, wherein the antigenic component is within the internal compartment.

11. The vaccine composition of any one of claims 8-10, wherein the antigenic component is selected from the group consisting of bovine antigens, caprine antigens, porcine antigens, poultry antigens, equine antigens, canine antigens, equine antigens and feline antigens.

12. The vaccine composition of any one of claims 8-11, wherein the immunostimulatory oligonucleotide comprises a CpG oligodeoxyribonucleotide.

13. The vaccine composition of claim 9, wherein the antigenic component comprises IBR, BVDV-1, and BVDV-2, and wherein the vaccine composition is essentially saponin-free.

14. A vaccine composition for use in a method of inducing an immune response against Bovine viral diarrhea virus (BVDV) in a bovine comprising administering to said bovine the vaccine composition according to claim 13.

15. The vaccine composition for use in method of claim 14, wherein said immune response is induced without an accompanying fever.

16. The vaccine composition of claim 12, wherein the antigen comprises a poultry antigen.

17. The vaccine composition of claim 16, wherein the antigen is profilin.

18. A vaccine composition for use in a method of prevention of *Eimeria* oocyst shedding in a poultry animal infected with *Eimeria,* comprising administering to said animal the vaccine composition of claim 17 prior to said infection.

19. The vaccine composition of claim 11, wherein the antigenic component comprises an ssRNA virus, and wherein the vaccine composition is substantially free of CpG oligodeoxyribonucleotide.

20. The vaccine of claim 19, wherein the ssRNA virus is an influenza virus.

21. The vaccine of claim 20, wherein the influenza virus is an inactivated Swine Influenza Virus (SIV).

22. A method of preparing the liposome of claim 1, the method comprising:
a) dissolving in an organic solvent the quaternary ammonium compound, the sterol, the phospholipid, and the glycolipid of formula I; 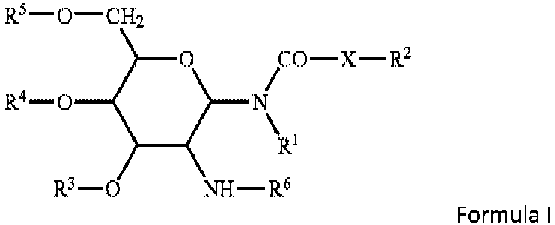 wherein, R¹ is hydrogen, or a saturated alkyl radical having up to 20 carbon atoms; X is -CH₂-, -O- or -NH-; R² is hydrogen, or a saturated or unsaturated alkyl radical having up to 20 carbon atoms; R⁴, R⁴, and R⁵ are independently hydrogen, -SO₄²⁻, -PO₄²⁻, -COC₁₋₁₀ alkyl; R⁶ is L-alanyl, L-alpha-aminobutyl, L-arginyl, L-asparginyl, L-aspartyl, L-cysteinyl, L-glutamyl, L-glycyl, L-histidyl, L-hydroxyprolyl, L-isoleucyl, L-leucyl, L-lysyl, L-methionyl, L-ornithinyl, L-phenyalany, L-prolyl, L-seryl, L-threonyl, L-tyrosyl, L-tryptophanyl, and L-valyl or their D-isomers;
b) removing the organic solvent and forming a film;
c) rehydrating the film in an aqueous solvent thereby forming a rehydrated composition;
d) microfluidizing the rehydrated composition.

23. The method of claim 22, wherein the aqueous solvent comprises an immunostimulatory oligonucleotide.

## Patentansprüche

1. Im Wesentlichen Saponin-freies Liposom, das eine äußere Lipiddoppelschichtmembran und ein inneres Kompartiment umfasst, wobei die äußere Membran umfasst:
a) eine quarternäre Ammoniumverbindung, die aus vier Alkylketten besteht, von denen zwei C10-C20-Alkye sind und die restlichen zwei C1-C4-Alkyle sind;
b) ein Sterol, ausgewählt aus der Gruppe, bestehend aus β-Sitosterol, Stigmasterol, Ergosterol, Ergocalciferol und Cholesterol;
c) ein Phospholipid und
d) ein Glycolipid der Formel I: 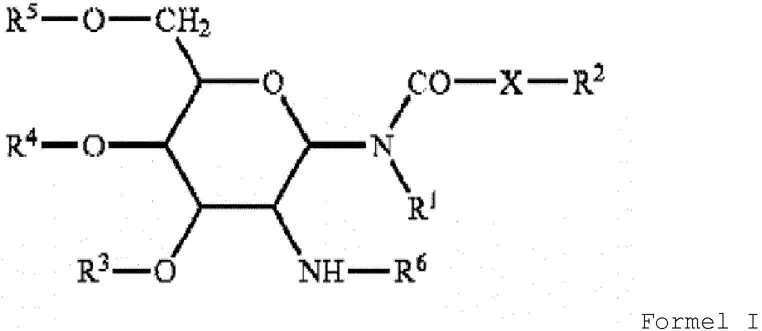
wobei R¹ Wasserstoff oder ein gesättigter Alkylrest mit bis zu 20 Kohlenstoffatomen ist; X -CH₂-, -O- oder -NH- ist; R² Wasserstoff oder ein gesättigter oder ungesättigter Alkylrest mit bis zu 20 Kohlenstoffatomen ist; R³, R⁴ und R⁵ unabhängig Wasserstoff, -SO₄²-, -PO₄³⁻, -COC₁₋₁₀-Alkyl sind; R⁶ L-Alanyl, L-alpha-Aminobutyl, L-Arginyl, L-Asparaginyl, L-Aspartyl, L-Cysteinyl, L-Glutamyl, L-Glycyl, L-Histidyl, L-Hydroxyprolyl, L-Isoleucyl, L-Leucyl, L-Lysyl, L-Methionyl, L-Ornithinyl, L-Phenylalanyl, L-Prolyl, L-Seryl, L-Threonyl, L-Tyrosyl, L-Tryptophanyl und L-Valyl oder ihre D-Isomeren ist.

2. Liposom nach Anspruch 1, wobei das Liposom Saponin-frei ist.

3. Liposom nach Anspruch 1 oder 2, wobei die quaternäre Ammoniumverbindung DDA (Dimethyldioctadecylammonium) ist, das Sterol Cholesterol ist und das Glycolipid N-(2-Desoxy-2-L-leucylamino-β-D-glucopyranosyl)-N-octadecyldodecanoylamid oder ein Salz davon ist.

4. Liposom nach einem der Ansprüche 1-3, das außerdem im inneren Kompartiment ein immunstimulatorisches Oligonucleotid, ausgewählt aus der Gruppe, bestehend aus einem immunstimulatorischen Ribonucleotid, einem CpG-Oligodesoxyribonucleotid und einer Kombination davon, umfasst.

5. Liposom nach Anspruch 4, wobei das immunstimulatorische Oligonucleotid eine von SEQ ID NOs 1-14 ist.

6. Adjuvans-Formulierung, die das Liposom gemäß einem der Ansprüche 1-5 umfasst.

7. Adjuvans-Zusammensetzung nach Anspruch 6, wobei die Adjuvans-Formulierung im Wesentlichen Saponin-frei ist.

8. Impfstoffzusammensetzung, umfassend eine wirksame Menge einer antigenen Komponente und die Adjuvans-Formulierung nach Anspruch 6 oder Anspruch 7.

9. Impfstoffzusammensetzung nach Anspruch 8, wobei die Impfstoffzusammensetzung im Wesentlichen Saponin-frei ist.

10. Impfstoffzusammensetzung nach Anspruch 8 oder 9, wobei die antigene Komponente in dem inneren Kompartiment ist.

11. Impfstoffzusammensetzung nach einem der Ansprüche 8-10, wobei die antigene Komponente aus der Gruppe, bestehend aus Rinderantigenen, Ziegenantigenen, Schweineantigenen, Geflügelantigenen, Pferdeantigenen, Hundeantigenen, Pferdeantigenen und Katzenantigenen, ausgewählt ist.

12. Impfstoffzusammensetzung nach einem der Ansprüche 8-11, wobei das immunstimulatorische Oligonucleotid ein CpG-Oligodesoxyribonucleotid umfasst.

13. Impfstoffzusammensetzung nach Anspruch 9, wobei die antigene Komponente IBR, BVDV-1 und BVDV-2 umfasst und wobei die Impfstoffzusammensetzung im Wesentlichen Saponin-frei ist.

14. Impfstoffzusammensetzung zur Verwendung in einem Verfahren zum Induzieren einer Immunantwort gegen das Virus der viralen Rinderdiarrhoe (BVDV) in einem Rind, das Verabreichen der Impfstoffzusammensetzung gemäß Anspruch 13 an das Rind umfasst.

15. Impfstoffzusammensetzung zur Verwendung im Verfahren nach Anspruch 14, wobei die Immunantwort ohne begleitendes Fieber induziert wird.

16. Impfstoffzusammensetzung nach Anspruch 12, wobei das Antigen ein Geflügelantigen umfasst.

17. Impfstoffzusammensetzung nach Anspruch 16, wobei das Antigen Profilin ist.

18. Impfstoffzusammensetzung zur Verwendung in einem Verfahren zur Prävention von Eimeria-Oozyten-Abstossung bei einem mit Eimeria infizierten Geflügeltier, das Verabreichen der Impfstoffzusammensetzung nach Anspruch 17 vor der Infektion an das Tier umfasst.

19. Impfstoffzusammensetzung nach Anspruch 11, wobei die antigene Komponente ein ssRNA-Virus umfasst und wobei die Impfstoffzusammensetzung im Wesentlichen frei von CpG-Oligodesoxyribonucleotid.

20. Impfstoff nach Anspruch 19, wobei das ssRNA-Virus ein Influenza-Virus ist.

21. Impfstoff nach Anspruch 20, wobei das Influenzavirus ein inaktiviertes Schweineinfluenzavirus (SIV) ist.

22. Verfahren zur Herstellung des Liposom nach Anspruch 1, wobei das Verfahren umfasst
a) Lösen der quaternären Ammoniumverbindung, des Sterols, des Phospholipids und des Glycolipids der Formel I: 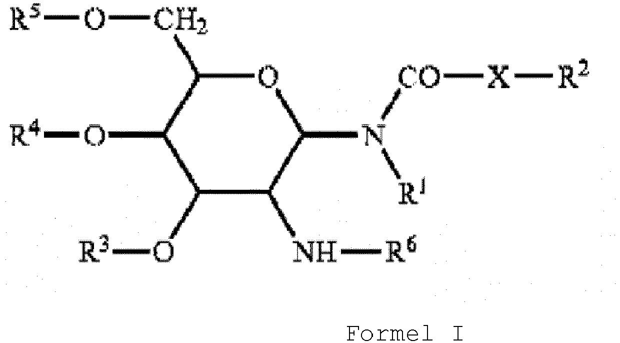 wobei R¹ Wasserstoff oder ein gesättigter Alkylrest mit bis zu 20 Kohlenstoffatomen ist; X -CH₂-, -O- oder -NH- ist; R² Wasserstoff oder ein gesättigter oder ungesättigter Alkylrest mit bis zu 20 Kohlenstoffatomen ist; R³, R⁴ und R⁵ unabhängig Wasserstoff, -SO₄²⁻, -PO₄³⁻, -COC₁₋₁₀-Alkyl
sind; R⁶ L-Alanyl, L-alpha-Aminobutyl, L-Arginyl, L-Asparaginyl, L-Aspartyl, L-Cysteinyl, L-Glutamyl, L-Glycyl, L-Histidyl, L-Hydroxyprolyl, L-Isoleucyl, L-Leucyl, L-Lysyl, L-Methionyl, L-Ornithinyl, L-Phenylalanyl, L-Prolyl, L-Seryl, L-Threonyl, L-Tyrosyl, L-Tryptophanyl und L-Valyl oder ihre D-Isomeren ist, in einem organischen Lösungsmittel;
b) Entfernen des organischen Lösungsmittels und Bilden eines Films;
c) Rehydratisieren des Films in einem wässrigen Lösungsmittel, dadurch Bilden einer rehydratisierten Zusammensetzung;
d) Mikrofluidisieren der rehydratisierten Zusammensetzung.

23. Verfahren nach Anspruch 22, wobei das wässrige Lösungsmittel ein immunstimulatorisches Oligonucleotid umfasst.

## Revendications

1. Liposome essentiellement exempt de saponine comprenant une membrane bicouche lipidique externe et un compartiment interne, la membrane externe comprenant :
a) un composé d'ammonium quaternaire composé de quatre chaînes alkyle, dont deux sont des alkyles en C10 à C20 et les deux restantes sont des alkyles en C1 à C4 ;
b) un stérol sélectionné dans le groupe consistant en le β-sitostérol, le stigmastérol, l'ergostérol, l'ergocalciférol, et le cholestérol ;
c) un phospholipide ; et
d) un glycolipide de formule I : 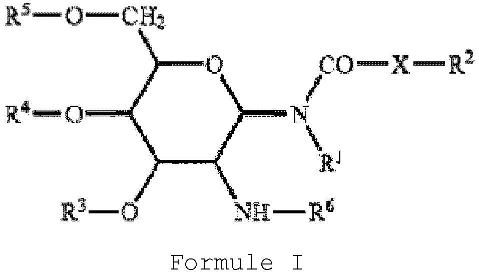
dans laquelle, R¹ est un hydrogène, ou un radical alkyle saturé ayant jusqu'à 20 atomes de carbone ; X est -CH₂-, -O- ou -NH- ; R² est un hydrogène, ou un radical alkyle saturé ou insaturé ayant jusqu'à 20 atomes de carbone ; R³, R⁴, et R⁵ sont indépendamment un hydrogène, -SO₄²⁻, -PO₄²⁻, un -CO-alkyle en C₁₋₁₀ ; R⁶ est un L-alanyle, un L-alpha-aminobutyle, un L-arginyle, un L-asparginyle, un L-aspartyle, un L-cystéinyle, un L-glutamyle, un L-glycyle, un L-histidyle, un L-hydroxyprolyle, un L-isoleucyle, un L-leucyle, un L-lysyle, un L-méthionyle, un L-ornithinyle, un L-phényalany, un L-prolyle, un L-séryle, un L-thréonyle, un L-tyrosyle, un L-tryptophanyle, et un L-valyle ou leurs isomères D.

2. Liposome selon la revendication 1, dans lequel ledit liposome est exempt de saponine.

3. Liposome selon la revendication 1 ou 2, dans lequel le composé d'ammonium quaternaire est le DDA (diméthyldioctadécylammonium), le stérol est le cholestérol, et le glycolipide est le N-(2-désoxy-2-L-leucylamino-β-D-glucopyranosyl)-N-octadécyldodécanoyl-amide ou un sel de celui-ci.

4. Liposome selon l'une des revendications 1 à 3, comprenant en outre, dans le compartiment interne, un oligonucléotide immunostimulateur sélectionné dans le groupe consistant en un ribonucléotide immunostimulateur, un oligodésoxy-ribonucléotide CpG, et une combinaison de ceux-ci.

5. Liposome selon la revendication 4, dans lequel ledit oligonucléotide immunostimulateur comprend l'une des SEQ ID NO 1 à 14.

6. Formulation d'adjuvant comprenant le liposome selon l'une des revendications 1 à 5.

7. Composition d'adjuvant selon la revendication 6, dans laquelle ladite formulation d'adjuvant est essentiellement exempte de saponine.

8. Composition de vaccin comprenant une quantité efficace d'un composant antigénique et la formulation d'adjuvant selon la revendication 6 ou la revendication 7.

9. Composition de vaccin selon la revendication 8, dans laquelle ladite composition de vaccin est essentiellement exempte de saponine.

10. Composition de vaccin selon la revendication 8 ou 9, dans laquelle le composant antigénique est à l'intérieur du compartiment interne.

11. Composition de vaccin selon l'une des revendications 8 à 10, dans laquelle le composant antigénique est sélectionné dans le groupe consistant en des antigènes bovins, des antigènes caprins, des antigènes porcins, des antigènes de volaille, des antigènes équins, des antigènes canins, des antigènes équins et des antigènes félins.

12. Composition de vaccin selon l'une des revendications 8 à 11, dans laquelle l'oligonucléotide immunostimulateur comprend un oligodésoxyribonucléotide CpG.

13. Composition de vaccin selon la revendication 9, dans laquelle le composant antigénique comprend l'IBR, le BVDV-1, et le BVDV-2, et dans laquelle la composition de vaccin est essentiellement exempte de saponine.

14. Composition de vaccin pour son utilisation dans un procédé d'induction d'une réponse immunitaire contre le virus de la diarrhée virale du bovin (BVDV) chez un bovin comprenant l'administration audit bovin de la composition de vaccin selon la revendication 13.

15. Composition de vaccin pour son utilisation dans le procédé selon la revendication 14, dans laquelle ladite réponse immunitaire est induite sans fièvre associée.

16. Composition de vaccin selon la revendication 12, dans laquelle l'antigène comprend un antigène de volaille.

17. Composition de vaccin selon la revendication 16, dans laquelle l'antigène est la profiline.

18. Composition de vaccin pour son utilisation dans un procédé de prévention de l'excrétion d'oocyste *d'Eimeria* chez un animal de type volaille infecté par *Eimeria,* comprenant l'administration audit animal de la composition de vaccin selon la revendication 17 avant ladite infection.

19. Composition de vaccin selon la revendication 11, dans laquelle le composant antigénique comprend un virus à ARNsb, et dans laquelle la composition de vaccin est sensiblement exempte d'oligodésoxyribonucléotide CpG.

20. Vaccin selon la revendication 19, dans lequel le virus à ARNsb est un virus de la grippe.

21. Vaccin selon la revendication 20, dans lequel le virus de la grippe est un virus de la grippe porcine (SIV) inactivé.

22. Procédé de préparation du liposome selon la revendication 1, le procédé comprenant :
a) la dissolution dans un solvant organique du composé d'ammonium quaternaire, du stérol, du phospholipide, et du glycolipide de formule I : 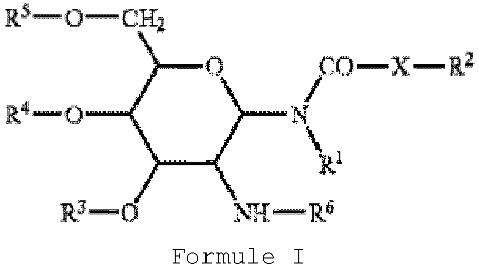 dans laquelle, R¹ est un hydrogène, ou un radical alkyle saturé ayant jusqu'à 20 atomes de carbone ; X est -CH₂-, -O- ou -NH- ; R² est un hydrogène, ou un radical alkyle saturé ou insaturé ayant jusqu'à 20 atomes de carbone ; R³, R⁴, et R⁵ sont indépendamment un hydrogène, -SO₄²⁻, -PO₄²⁻, un -CO-alkyle en C₁₋₁₀ ; R⁶ est un L-alanyle, un L-alpha-aminobutyle, un L-arginyle, un L-asparginyle, un L-aspartyle, un L-cystéinyle, un L-glutamyle, un L-glycyle, un L-histidyle, un L-hydroxyprolyle, un L-isoleucyle, un L-leucyle, un L-lysyle, un L-méthionyle, un L-ornithinyle, un L-phényalany, un L-prolyle, un L-séryle, un L-thréonyle, un L-tyrosyle, un L-tryptophanyle, et un L-valyle ou leurs isomères D ;
b) l'élimination du solvant organique et la formation d'un film ;
c) la réhydratation du film dans un solvant aqueux pour ainsi former une composition réhydratée ;
d) la microfluidisation de la composition réhydratée.

23. Procédé selon la revendication 22, dans lequel le solvant aqueux comprend un oligonucléotide immunostimulateur.
